# EUROPEAN PATENT APPLICATION

(11) **EP 4 588 476 A1**
(43) Date of publication of application: **23.07.2025**
(21) Application number: 24217100.7
(22) Date of filing: 28.08.2018
(51) Int. Cl.: A61K 31/445, A61P 9/04, A61P 43/00

(54) **METHODS OF ENHANCING AND/OR STABILIZING CARDIAC FUNCTION IN PATIENTS WITH FABRY DISEASE**

(30) Priority: 28.08.2017 US 201762550984 P
(62) Divisional of application: 22194251.9
(71) Applicant: Amicus Therapeutics, Inc., Philadelphia, PA 19104 (US)
(72) Inventor: CASTELLI, Jeff, Philadelphia,, 19104 (US); BARTH, Jay, Philadelphia,, 19104 (US); SKUBAN, Nina, Philadelphia,, 19104 (US)
(74) Representative: Hoffmann Eitle

(57) **Abstract**

Provided are methods for the treatment of Fabry disease in a patient. Certain methods relate to the treatment of ERT-experienced or ERT-naïve Fabry patients. Certain methods comprise administering to the patient about 100 mg to about 150 mg free base equivalent of migalastat for enhancing and/or stabilizing cardiac function.

## Description

### CROSS-REFERENCE TO RELATED APPLICATIONS

This application claims priority to U.S. Provisional Application Serial No. 62/550,984 filed on August 28, 2017, which is incorporated by reference herein in its entirety.

### TECHNICAL FIELD

Principles and embodiments of the present invention relate generally to the use of pharmacological chaperones for the treatment of lysosomal storage disorders, particularly the use of migalastat for the treatment of Fabry disease.

### BACKGROUND

Fabry disease is a progressive, X-linked inborn error of glycosphingolipid metabolism caused by a deficiency in the lysosomal enzyme α-galactosidase A (α-Gal A) as a result of mutations in the α-Gal A gene (GLA). Despite being an X-linked disorder, females can express varying degrees of clinical manifestations. Fabry is a rare disease with incidence estimated between 1 in 40,000 males to 1 in 117,000 in the general population. Moreover, there are variants of later onset phenotype of Fabry disease that can be under-diagnosed, as they do not present with classical signs and symptoms. This, and newborn screening for Fabry disease, suggests that the actual incidence of Fabry disease can be higher than currently estimated.

Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. Gradual deterioration of renal function and the development of azotemia, due to glycosphingolipid deposition, usually occur in the third to fifth decades of life, but can occur as early as in the second decade. Renal lesions are found in both hemizygous (male) and heterozygous (female) patients.

Cardiac disease as a result of Fabry disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

One approved therapy for treating Fabry disease is enzyme replacement therapy (ERT), which typically involves intravenous, infusion of a purified form of the corresponding wild-type protein. Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). While ERT is effective in many settings, the treatment also has limitations. ERT has not been demonstrated to decrease the risk of stroke, cardiac muscle responds slowly, and GL-3 elimination from some of the cell types of the kidneys is limited. Some patients also develop immune reactions to ERT.

Accordingly, there remains a need for therapies for the treatment of Fabry disease, especially for enhancing the cardiac function.

### SUMMARY

Various aspects of the present invention relate to the treatment of Fabry disease in ERT-naïve and ERT-experienced patients using migalastat. Such treatment can include enhancing and/or stabilizing cardiac function, such as increasing and/or stabilizing midwall fractional shortening (MWFS).

One aspect of the present invention pertains to a method of enhancing cardiac function in a patient having Fabry disease, the method comprising administering to the patient a formulation comprising an effective amount of migalastat or salt thereof every other day for enhancing the patient's cardiac function, wherein the effective amount is about 100 mg to about 150 mg free base equivalent (FBE).

In one or more embodiments, enhancing cardiac function comprises enhancing left ventricular systolic function.

In one or more embodiments, the patient has impaired MWFS prior to initiating administration of the migalastat or salt thereof.

In one or more embodiments, the patient has left ventricular hypertrophy (LVH) prior to initiating administration of the migalastat or salt thereof.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

In one or more embodiments, the migalastat or salt thereof is administered for at least 12 months.

In one or more embodiments, the migalastat or salt thereof is administered for at least 24 months.

In one or more embodiments, the patient is an ERT-naïve patient.

In one or more embodiments, the administration of migalastat or a salt thereof provides an average increase in MWFS in a group of ERT-naïve patients with impaired MWFS of at least about 1% after 24 months of administration of migalastat or a salt thereof.

In one or more embodiments, the patient is an ERT-experienced patient.

Another aspect of the present invention pertains to a method of increasing MWFS in a patient having Fabry disease, the method comprising administering to the patient a formulation comprising an effective amount of migalastat or salt thereof every other day for increasing the patient's MWFS, wherein the effective amount is about 100 mg to about 150 mg FBE.

In one or more embodiments, the patient has impaired MWFS prior to initiating administration of the migalastat or salt thereof.

In one or more embodiments, the patient has LVH prior to initiating administration of the migalastat or salt thereof.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

In one or more embodiments, the migalastat or salt thereof is administered for at least 12 months.

In one or more embodiments, the migalastat or salt thereof is administered for at least 24 months.

In one or more embodiments, the patient is an ERT-naïve patient.

In one or more embodiments, the administration of migalastat or a salt thereof provides an average increase in MWFS in a group of ERT-naïve patients with impaired MWFS of at least about 1% after 24 months of administration of migalastat or a salt thereof.

In one or more embodiments, the patient is an ERT-experienced patient.

Another aspect of the present invention pertains to a method of normalizing MWFS in a patient having Fabry disease and impaired MWFS, the method comprising administering to the patient a formulation comprising an effective amount of migalastat or salt thereof every other day for normalizing the patient's MWFS, wherein the effective amount is about 100 mg to about 150 mg FBE.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

In one or more embodiments, the migalastat or salt thereof is administered for at least 12 months.

In one or more embodiments, the migalastat or salt thereof is administered for at least 24 months.

In one or more embodiments, the patient is an ERT-naïve patient.

In one or more embodiments, the administration of migalastat or a salt thereof provides an average increase in MWFS in a group of ERT-naïve patients with impaired MWFS of at least about 1% after 24 months of administration of migalastat or a salt thereof.

Another aspect of the present invention pertains to a method of stabilizing MWFS in a patient having Fabry disease, the method comprising administering to the patient a formulation comprising an effective amount of migalastat or salt thereof every other day for stabilizing the patient's MWFS, wherein the effective amount is about 100 mg to about 150 mg FBE.

In one or more embodiments, the patient has impaired MWFS prior to initiating administration of the migalastat or salt thereof.

In one or more embodiments, the patient has LVH prior to initiating administration of the migalastat or salt thereof.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

In one or more embodiments, the migalastat or salt thereof is administered for at least 12 months.

In one or more embodiments, the migalastat or salt thereof is administered for at least 30 months.

In one or more embodiments, the patient is an ERT-experienced patient.

In one or more embodiments, the administration of migalastat or a salt thereof provides an average change in MWFS in a group of ERT-experienced patients with impaired MWFS of greater than about -0.5% after 30 months of administration of migalastat or a salt thereof.

### BRIEF DESCRIPTION OF THE DRAWINGS

Further features of the present invention will become apparent from the following written description and the accompanying figures, in which:
FIGS. 1A-E show the full DNA sequence of the human wild-type GLA gene (SEQ ID NO: 1);
FIG. 2 shows the wild-type α-Gal A protein (SEQ ID NO: 2); and
FIG. 3 shows the nucleic acid sequence encoding the wild-type α-Gal A protein (SEQ ID NO: 3).

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

Various aspects of the present invention pertain to dosing regimens for the administration of pharmacological chaperones such as migalastat for the treatment of Fabry disease. In one or more embodiments, the dosing regimens of migalastat enhance one or more cardiac parameters of a patient.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "Fabry disease" refers to an X-linked inborn error of glycosphingolipid catabolism due to deficient lysosomal α-Gal A activity. This defect causes accumulation of the substrate globotriaosylceramide ("GL-3", also known as Gb3 or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gb₃").

The term "atypical Fabry disease" refers to patients with primarily cardiac manifestations of the α-Gal A deficiency, namely progressive GL-3 accumulation in myocardial cells that leads to significant enlargement of the heart, particularly the left ventricle.

A "carrier" is a female who has one X chromosome with a defective α-Gal A gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

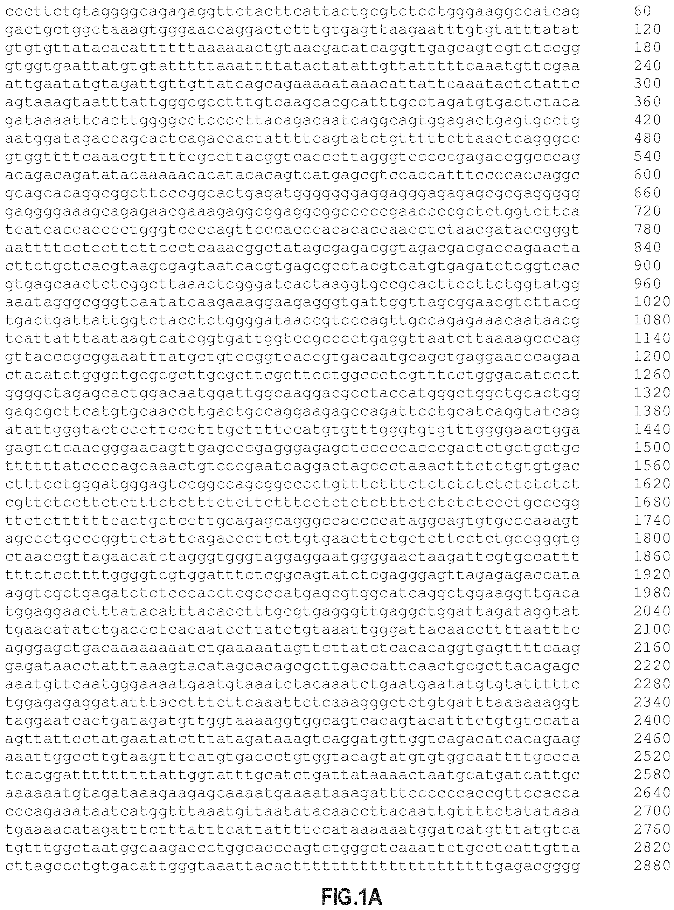

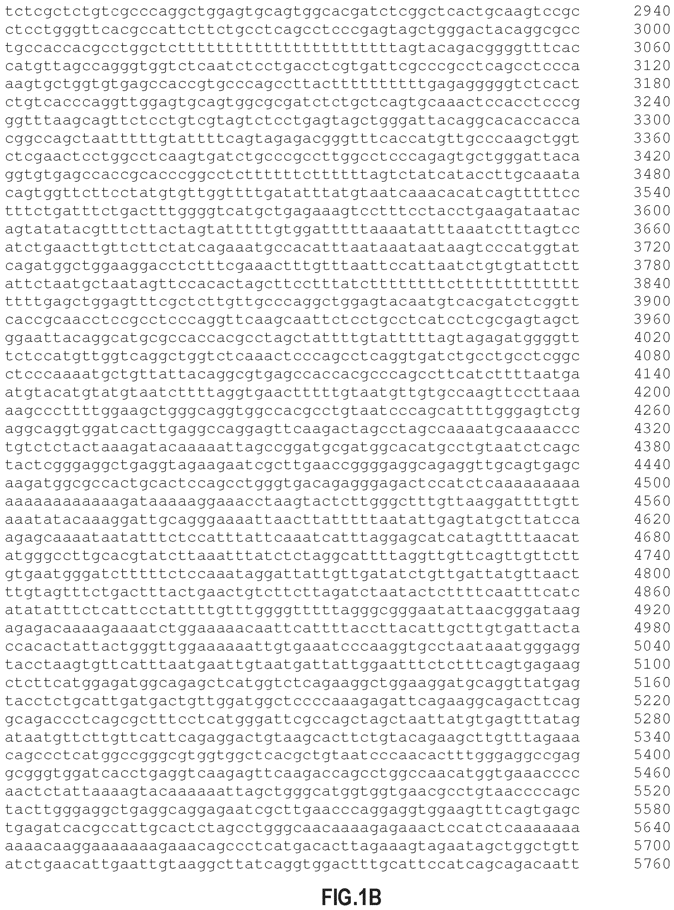

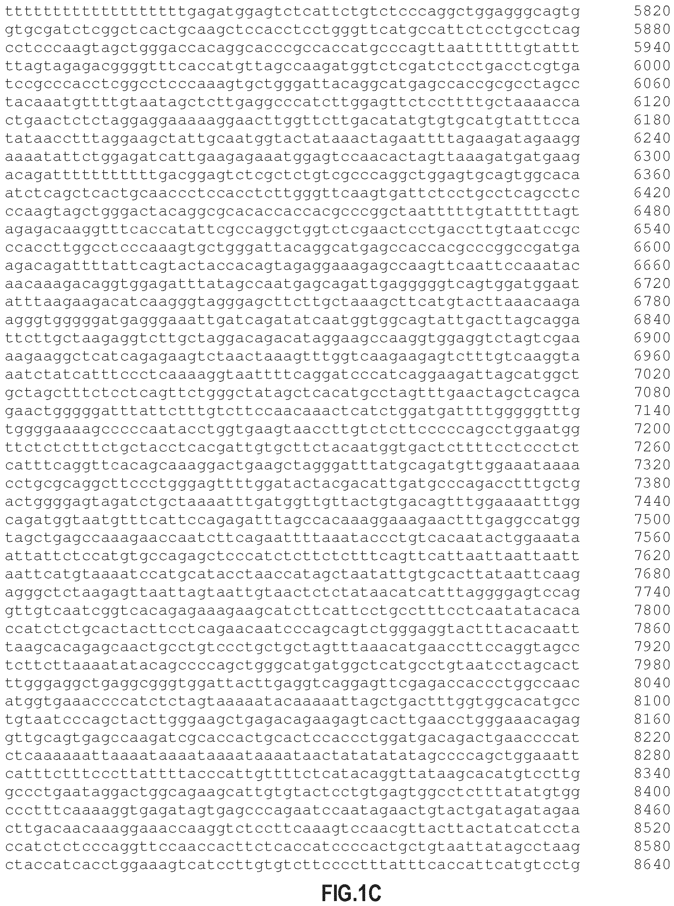

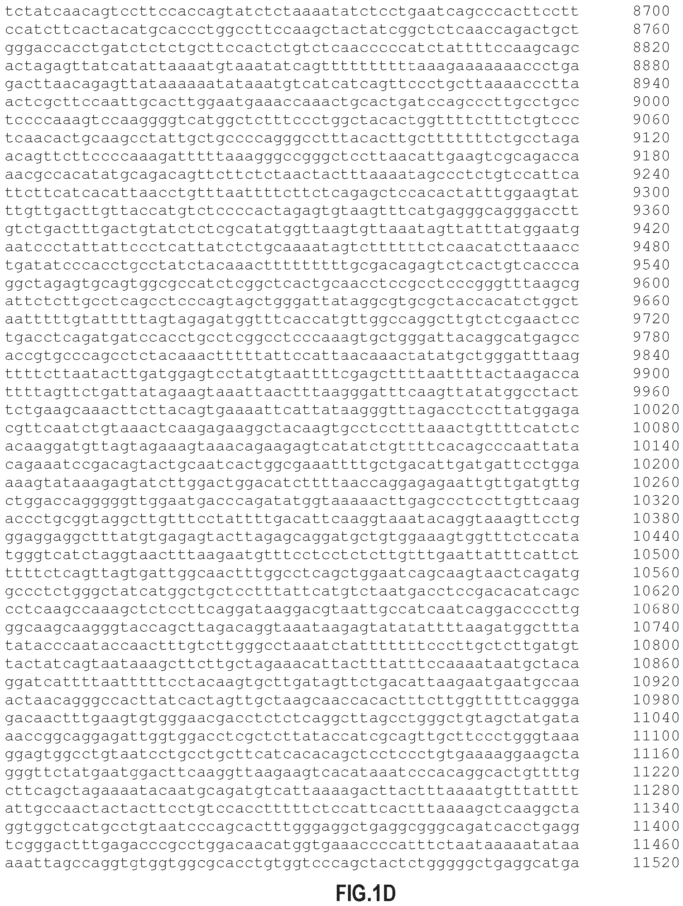

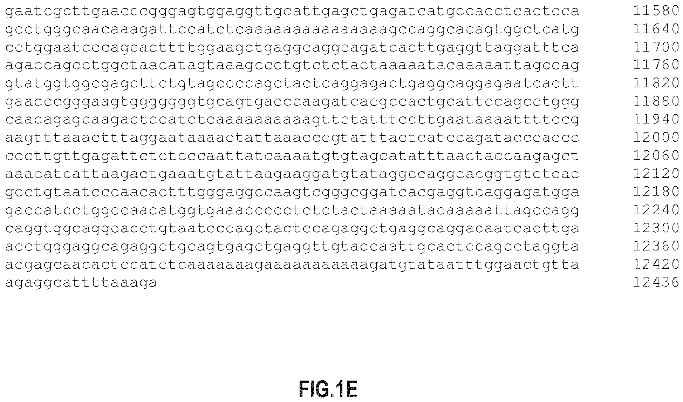

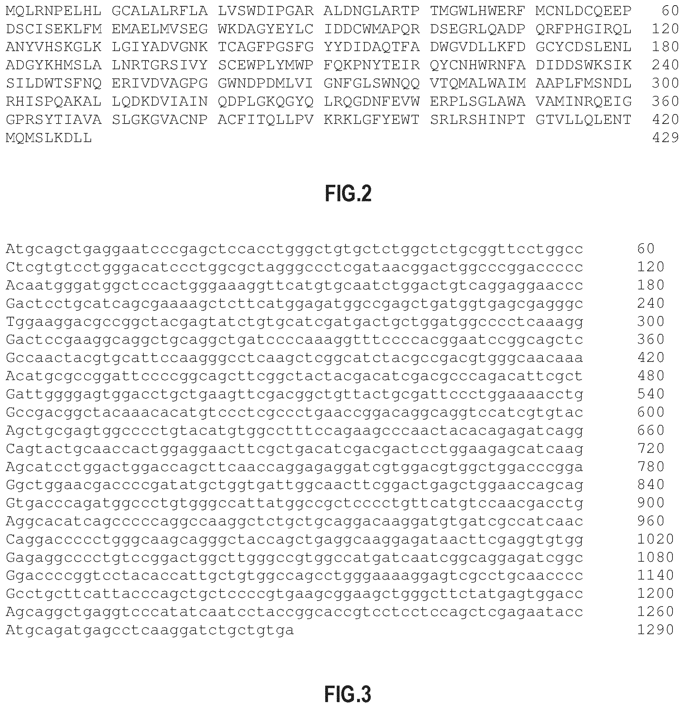

Human α-galactosidase A (α-Gal A) refers to an enzyme encoded by the human GLA gene. The full DNA sequence of α-Gal A, including introns and exons, is available in GenBank Accession No. X14448.1 and shown in FIG. 1A-E (SEQ ID NO: 1). The human α-Gal A enzyme consists of 429 amino acids and is available in GenBank Accession Nos. X14448.1 and U78027.1 and shown in FIG.2 (SEQ ID NO: 2). The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the endoplasmic reticulum (ER). The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions.

As used herein in one embodiment, the term "mutant α-Gal A" includes an α-Gal A which has a mutation in the gene encoding α-Gal A which results in the inability of the enzyme to achieve a stable conformation under the conditions normally present in the ER. The failure to achieve a stable conformation results in a substantial amount of the enzyme being degraded, rather than being transported to the lysosome.

As used herein, the term "pharmacological chaperone" ("PC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the ER to another cellular location, preferably a native cellular location, *i.e.,* prevents ER-associated degradation of the protein; (iii) prevents aggregation of misfolded proteins; and/or (iv) restores or enhances at least partial wild-type function and/or activity to the protein. A compound that specifically binds to *e.g.,* α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, *i.e.,* chemical chaperones. In one or more embodiments of the present invention, the PC may be a reversible competitive inhibitor. In one embodiment, the PC is migalastat or a salt thereof. In another embodiment, the PC is migalastat free base *(e.g.,* 123 mg of migalastat free base). In yet another embodiment, the PC is a salt of migalastat *(e.g.,* 150 mg of migalastat HCl).

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, *i.e.,* competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, *e.g.,* α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, *e.g*., co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, *e.g.,* at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "α-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder (LSD), such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-GB3 and those disclosed in US Patent Application Publication No. U.S. 2010/0113517, which is hereby incorporated by reference in its entirety.

Non-limiting examples of improvements in surrogate markers for Fabry disease disclosed in U.S. 2010/0113517 include increases in α-Gal A levels or activity in cells (*e.g.,* fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb₃; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; improvements in renal function; mitigation of hypohidrosis; the absence of angiokeratomas; and improvements in hearing abnormalities such as high frequency sensorineural hearing loss progressive hearing loss, sudden deafness, or tinnitus. Improvements in neurological symptoms include prevention of transient ischemic attack (TIA) or stroke; and amelioration of neuropathic pain manifesting itself as acroparaesthesia (burning or tingling in extremities). Another type of clinical marker that can be assessed for Fabry disease is the prevalence of deleterious cardiovascular manifestations.

"Midwall fractional shortening" or "MWFS" is a measure of systolic function that identifies hypertensive patients who have evidence of target-organ damage, impaired contractile reserve, and increased mortality.

The term "cardiac function" refers to the performance of a patient's heart. For example, one assessment of cardiac function is left ventricular systolic function, which refers to the emptying characteristics of the left heart. Left ventricular systolic function can be evaluated in several ways, including, but not limited to, left ventricular ejection fraction (LVEF), endocardial fractional shortening (EFS) and MWFS.

As used herein, the phrase "stabilizing cardiac function" and similar terms refer to reducing or arresting the decline in cardiac function and/or restoring cardiac function. As untreated Fabry patients are expected to have significant decreases in cardiac function over time, enhancements in the rate of cardiac function deterioration and/or enhancements in cardiac function demonstrate a benefit of migalastat therapy as described herein. In various embodiments, stabilizing cardiac function includes stabilizing MWFS. "Stabilizing MWFS" likewise refers to reducing or arresting the decline in MWFS.

The term "enhancing cardiac function" refers to a beneficial change in at least one parameter used to evaluate cardiac function. If a patient's parameter is at the lower end of a normal range or is below the normal range for that parameter, than a beneficial change in that parameter is an increase in that parameter. For example, an increase in MWFS for a patient having a low MWFS is an enhancement in that parameter. Similarly, if a patient's parameter is at the upper end of a normal range or is above the normal range for that parameter, than a beneficial change in that parameter is a decrease in that parameter. In an aspect, "enhancing cardiac function" comprises one or more of (i) improving left ventricular function, (ii) improving fractional shortening, (iii) improving ejection fraction, (iv) reducing end-diastolic volume, and (v) normalizing of heart geometry.

As used herein, the term "impaired MWFS" refers to a patient having an MWFS below the normal range. The normal range of MWFS for a female is at least 15% and the normal range of MWFS for a male is at least 14%. Thus, impaired MWFS for a female patient is <15% and impaired MWFS for a male patient is <14%.

As used herein, the term "normalizing MWFS" refers to increasing the MWFS of a patient from an impaired MWFS to within the normal range. Thus, normalizing MWFS for a female patient is increasing MWFS from <15% to at least 15%, and normalizing MWFS for a male patient is increasing MWFS from <14% to at least 14%.

As used herein, the term "left ventricular hypertrophy" or "LVH" refers to a patient having a left ventricular mass index (LVMi) above the normal range of 43-95 g/m² for females and 49-115 g/m² for males. Thus, LVH refers to a LVMi > 95 g/m² for females or > 115 g/m² for males.

The dose that achieves one or more of the aforementioned responses is a "therapeutically effective dose."

The phrase "pharmaceutically acceptable" refers to molecular entities and compositions that are physiologically tolerable and do not typically produce untoward reactions when administered to a human. In some embodiments, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

As used herein, the term "isolated" means that the referenced material is removed from the environment in which it is normally found. Thus, an isolated biological material can be free of cellular components, *i.e.,* components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, *e.g*., suffering from enzyme insufficiency. The introduced enzyme may be a purified, recombinant enzyme produced *in vitro,* or protein purified from isolated tissue or fluid, such as, *e.g.,* placenta or animal milk, or from plants.

The term "ERT-naïve patient" refers to a Fabry patient that has never received ERT or has not received ERT for at least 6 months prior to initiating migalastat therapy.

The term "ERT-experienced patient" refers to a Fabry patient that was receiving ERT immediately prior to initiating migalastat therapy. In some embodiments, the ERT-experienced patient has received at least 12 months of ERT immediately prior to initiating migalastat therapy.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts are expected to have different conversion factors, depending on the molecular weight of the salt.

The term "migalastat" encompasses migalastat free base or a pharmaceutically acceptable salt thereof (e.g., migalastat HCl), unless specifically indicated to the contrary.

The terms "mutation" and "variant" (e.g., as in "amenable mutation or variant") refer to a change in the nucleotide sequence of a gene or a chromosome. The two terms referred herein are typically used together - *e.g.,* as in "mutation or variant"- referring to the change in nucleotide sequence stated in the previous sentence. If only one of the two terms is recited for some reason, the missing term was intended to be included and one should understand as such. Furthermore, the terms "amenable mutation" and "amenable variant" refer to a mutation or variant that is amenable to PC therapy, *e.g.,* a mutation that is amenable to migalastat therapy. A particular type of amenable mutation or variant is a "HEK assay amenable mutation or variant", which is a mutation or variant that is determined to be amenable to migalastat therapy according to the criteria in the *in vitro* HEK assay described herein and in U.S. Patent No. 8,592,362, which is hereby incorporated by reference in its entirety.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

### Fabry Disease

Fabry disease is a rare, progressive and devastating X-linked LSD. Mutations in the GLA gene result in a deficiency of the lysosomal enzyme, α-Gal A, which is required for glycosphingolipid metabolism. Beginning early in life, the reduction in α-Gal A activity results in an accumulation of glycosphingolipids, including GL-3 and plasma lyso-Gb3, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1,000 Fabry disease-causing GLA mutations have been identified. Approximately 60% are missense mutations, resulting in single amino acid substitutions in the α-Gal A enzyme. Missense GLA mutations often result in the production of abnormally folded and unstable forms of α-Gal A and the majority are associated with the classic phenotype. Normal cellular quality control mechanisms in the ER block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-Gal A levels. The majority of currently treated patients are referred to as classic Fabry patients, most of whom are males. These patients experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as later-onset Fabry disease, tend to have higher residual α-Gal A levels than classic Fabry patients. Individuals with later-onset Fabry disease typically first experience disease symptoms in adulthood, and often have disease symptoms focused on a single organ, such as enlargement of the left ventricle or progressive kidney failure. In addition, later-onset Fabry disease may also present in the form of strokes of unknown cause.

Fabry patients have progressive kidney impairment, and untreated patients exhibit end-stage renal impairment by the fifth decade of life. Deficiency in α-Gal A activity leads to accumulation of GL-3 and related glycosphingolipids in many cell types including cells in the kidney. GL-3 accumulates in podocytes, epithelial cells and the tubular cells of the distal tubule and loop of Henle. Impairment in kidney function can manifest as proteinuria and reduced glomerular filtration rate.

Because Fabry disease is rare, involves multiple organs, has a wide age range of onset, and is heterogeneous, proper diagnosis is a challenge. Awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

In one or more embodiments, mutant forms of α-Gal A are considered to be amenable to migalastat are defined as showing a relative increase (+10 µM migalastat) of ≥1.20-fold and an absolute increase (+ 10 µM migalastat) of ≥ 3.0% wild-type (WT) when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations are also referred to herein as "HEK assay amenable" mutations.

Previous screening methods have been provided that assess enzyme enhancement prior to the initiation of treatment. For example, an assay using HEK-293 cells has been utilized in clinical trials to predict whether a given mutation will be responsive to pharmacological chaperone (e.g., migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (e.g., migalastat) using these methods are listed in U.S. Patent No. 8,592,362.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582 all incorporated herein by reference). In particular, administration of small molecule derivatives of glucose and galactose, which are specific, selective competitive inhibitors for several target lysosomal enzymes, effectively increased the stability of the enzymes in cells *in vitro* and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the ER (Ishii et al., Biochem. Biophys. Res. Comm. 1996; 220: 812-815), the enzyme protein is retarded in the normal transport pathway (ER→Golgi apparatus→endosomes→lysosome) and prematurely degraded. Therefore, a compound which binds to and increases the stability of a mutant enzyme, may serve as a "chaperone" for the enzyme and increase the amount that can exit the ER and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the ER and be trafficked to lysosomes.

In one or more embodiments, the pharmacological chaperone comprises migalastat or a salt thereof. The compound migalastat, also known as 1-deoxygalactonojirimycin (1-DGJ) or (2R,3S,4R,5S)-2-(hydroxymethyl) piperdine-3,4,5-triol is a compound having the following chemical formula:

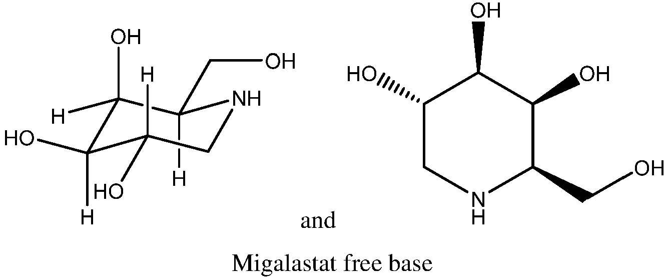

As discussed herein, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

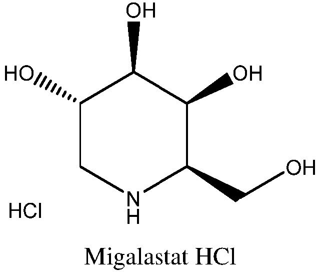

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type α-Gal A and specific mutant forms of α-Gal A, the genotypes of which are referred to as HEK assay amenable mutations. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates. Approximately 30-50% of patients with Fabry disease have HEK assay amenable mutations; the majority of which are associated with the classic phenotype of the disease.

HEK assay amenable mutations include at least those mutations listed in a pharmacological reference table *(e.g.,* the ones recited in the U.S. or International Product labels for a migalastat product such as GALAFOLD^{®}). As used herein, "pharmacological reference table" refers to any publicly accessible written or electronic record, included in either the product label within the packaging of a migalastat product (e.g., GALAFOLD^{®}) or in a website accessible by health care providers, that conveys whether a particular mutation or variant is responsive to migalastat (e.g., GALAFOLD^{®}) PC therapy, and is not necessarily limited to written records presented in tabular form. In one embodiment of the present invention, a "pharmacological reference table" thus refers to any depository of information that includes one or more amenable mutations or variants. An exemplary pharmacological reference table for HEK assay amenable mutations can be found in the summary of product characteristics and/or prescribing information for GALAFOLD^{®} in various countries in which GALAFOLD^{®} is approved for use, or at a website such as www.galafoldamenabilitytable.com or www.fabrygenevariantsearch.com, each of which is hereby incorporated by reference in its entirety.

An exemplary pharmacological reference table for HEK assay amenable mutations is provided in Table 1 below. In one or more embodiments, if a double mutation is present on the same chromosome (males and females), that patient is considered HEK assay amenable if the double mutation is present in one entry in Table 1 (e.g., D55V/Q57L). In some embodiments, if a double mutation is present on different chromosomes (only in females) that patient is considered HEK assay amenable if either one of the individual mutations is present in Table 1.

**Table 1**

| **Nucleotide change** | **Nucleotide change** | **Protein sequence change** |
|---|---|---|
| c.7C>G | c.C7G | L3V |
| c.8T>C | c.T8C | L3P |
| c.[11G>T; 620A>C] | c.G11T/A620C | R4M/Y207S |
| c.37G>A | c.G37A | A13T |
| c.37G>C | c.G37C | A13P |
| c.43G>A | c.G43A | A15T |
| c.44C>G | c.C44G | A15G |
| c.53T>G | c.T53G | F18C |
| c.58G>C | c.G58C | A20P |
| c.59C>A | c.C59A | A20D |
| c.70T>C or c.70T>A | c.T70C or c.T70A | W24R |
| c.70T>G | c.T70G | W24G |
| c.72G>C or c.72G>T | c.G72C or c.G72T | W24C |
| c.95T>C | c.T95C | L32P |
| c.97G>C | c.G97C | D33H |
| c.97G>T | c.G97T | D33Y |
| c.98A>G | c.A98G | D33G |
| c.100A>G | c.A100G | N34D |
| c.101A>C | c.A101C | N34T |
| c.101A>G | c.A101G | N34S |
| c.102T>G or c.102T>A | c.T102G or c.T102A | N34K |
| c.103G>C or c.103G>A | c.G103C or c.G103A | G35R |
| c.104G>A | c.G104A | G35E |
| c.104G>C | c.G104C | G35A |
| c.104G>T | c.G104T | G35V |
| c.107T>C | c.T107C | L36S |
| c.107T>G | c.T107G | L36W |
| c.108G>C or c.108G>T | c.G108C or c.G108T | L36F |
| c.109G>A | c.G109A | A37T |
| c.110C>T | c.C110T | A37V |
| c.122C>T | c.C122T | T41I |
| c.124A>C or c.124A>T | c.A124C or c.A124T | M42L |
| c.124A>G | c.A124G | M42V |
| c.125T>A | c.T125A | M42K |
| c.125T>C | c.T125C | M42T |
| c.125T>G | c.T125G | M42R |
| c.126G>A or c.126G>C or c.126G>T | c.G126A or c.G126C or c.G126T | M42I |
| c.137A>C | c.A137C | H46P |
| c.142G>C | c.G142C | E48Q |
| c.152T>A | c.T152A | M51K |
| c.153G>A or c.153G>T or c.153G>C | c.G153A or c.G153T or c.G153C | M51I |
| c.157A>G | c.A157G | N53D |
| c.[157A>C; 158A>T] | c.A157C/A158T | N53L |
| c.160C>T | c.C160T | L54F |
| c.161T>C | c.T161C | L54P |
| c.164A>G | c.A164G | D55G |
| c.164A>T | c.A164T | D55V |
| c.[164A>T; 170A>T] | c.A164T/A170T | D55V/Q57L |
| c.167G>T | c.G167T | C56F |
| c.167G>A | c.G167A | C56Y |
| c.170A>T | c.A170T | Q57L |
| c.175G>A | c.G175A | E59K |
| c.178C>A | c.C178A | P60T |
| c.178C>T | c.C178T | P60S |
| c.179C>T | c.C179T | P60L |
| c.196G>A | c.G196A | E66K |
| c.197A>G | c.A197G | E66G |
| c.207C>A or c.207C>G | c.C207A or c.C207G | F69L |
| c.214A>G | c.A214G | M72V |
| c.216G>A or c.216G>T or c.216G>C | c.G216A or c.G216T or c.G216C | M72I |
| c.218C>T | c.C218T | A73V |
| c.227T>C | c. T227C | M76T |
| c.239G>A | c.G239A | G80D |
| c.247G>A | c.G247A | D83N |
| c.253G>A | c.G253A | G85S |
| c.254G>A | c.G254A | G85D |
| c.[253G>A; 254G>A] | c.G253A/G254A | G85N |
| c.[253G>A; 254G>T; 255T>G] | c.G253A/G254T/T255G | G85M |
| c.261G>C or c.261G>T | c.G261C or c.G261T | E87D |
| c.263A>C | c.A263C | Y88S |
| c.265C>T | c.C265T | L89F |
| c.272T>C | c.T272C | I91T |
| c.288G>A or c.288G>T or c.288G>C | c.G288A or c.G288T or c.G288C | M96I |
| c.289G>C | c.G289C | A97P |
| c.290C>T | c.C290T | A97V |
| c.305C>T | c.C305T | S102L |
| c.311G>T | c.G311T | G104V |
| c.316C>T | c.C316T | L106F |
| c.322G>A | c.G322A | A108T |
| c.326A>G | c.A326G | D 109G |
| c.334C>G | c.C334G | R112G |
| c.335G>A | c.G335A | R112H |
| c.337T>A | c.T337A | F113I |
| c.337T>C or c.339T>A or c.339T>G | c.T337C or c.T339A or c.T339G | F113L |
| c.352C>T | c.C352T | R118C |
| c.361G>A | c.G361A | A121T |
| c.368A>G | c.A368G | Y123C |
| c.373C>T | c.C373T | H125Y |
| c.374A>T | c.A374T | H125L |
| c.376A>G | c.A376G | S126G |
| c.383G>A | c.G383A | G128E |
| c.399T>G | c.T399G | I133M |
| c.404C>T | c.C404T | A135V |
| c.408T>A or c.408T>G | c.T408A or c.T408G | D136E |
| c.416A>G | c.A416G | N139S |
| c.419A>C | c.A419C | K140T |
| c.427G>A | c.G427A | A143T |
| c.431G>A | c.G431A | G144D |
| c.431G>T | c.G431T | G144V |
| c.434T>C | c.T434C | F145S |
| c.436C>T | c.C436T | P146S |
| c.437C>G | c.C437G | P146R |
| c.454T>C | c.T454C | Y152H |
| c.455A>G | c.A455G | Y152C |
| c.466G>A | c.G466A | A156T |
| c.467C>T | c.C467T | A156V |
| c.471G>C or c.471G>T | c.G471C or c.G471T | Q157H |
| c.484T>G | c.T484G | W162G |
| c.493G>C | c.G493C | D165H |
| c.494A>G | c.A494G | D165G |
| c.[496C>G; 497T>G] | c.C496G/T497G | L166G |
| c.496C>G | c.C496G | L166V |
| c.496_497delinsTC | c.496_497delinsTC | L166S |
| c.499C>G | c.C499G | L167V |
| c.506T>C | c.T506C | F169S |
| c.511G>A | c.G511A | G171S |
| c.520T>C | c.T520C | C174R |
| c.520T>G | c.T520G | C174G |
| c.525C>G or c.525C>A | c.C525G or c.C525A | D175E |
| c.539T>G | c.T539G | L180W |
| c.540G>C | c.G540C | L180F |
| c.548G>C | c.G548C | G183A |
| c.548G>A | c.G548A | G183D |
| c.550T>A | c.T550A | Y184N |
| c.551A>G | c.A551G | Y184C |
| c.553A>G | c.A553G | K185E |
| c.559A>G | c.A559G | M187V |
| c.559_564dup | c.559_564dup | p.M187_S188dup |
| c.560T>C | c.T560C | M187T |
| c.561G>T or c.561G>A or c.561G>C | c.G561T or c.G561A or c.G561C | M187I |
| c.572T>A | c.T572A | L191Q |
| c.580A>G | c.A580G | T194A |
| c.581C>T | c.C581T | T194I |
| c.584G>T | c.G584T | G195V |
| c.586A>G | c.A586G | R196G |
| c.593T>C | c.T593C | I198T |
| c.595G>A | c.G595A | V199M |
| c.596T>C | c.T596C | V199A |
| c.596T>G | c.T596G | V199G |
| c.599A>G | c.A599G | Y200C |
| c.602C>T | c.C602T | S201F |
| c.602C>A | c.C602A | S201Y |
| c.608A>T | c.A608T | E203V |
| c.609G>C or c.609G>T | c.G609C or c.G609T | E203D |
| c.610T>G | c.T610G | W204G |
| c.613C>A | c.C613A | P205T |
| c.613C>T | c.C613T | P205S |
| c.614C>T | c.C614T | P205L |
| c.619T>C | c.T619C | Y207H |
| c.620A>C | c.A620C | Y207S |
| c.623T>G | c.T623G | M208R |
| c.628C>T | c.C628T | P210S |
| c.629C>T | c.C629T | P210L |
| c.638A>G | c.A638G | K213R |
| c.638A>T | c.A638T | K213M |
| c.640C>T | c.C640T | P214S |
| c.641C>T | c.C641T | P214L |
| c.643A>G | c.A643G | N215D |
| c.644A>G | c.A644G | N215S |
| c.644A>T | c.A644T | N215I |
| c.[644A>G; 937G>T] | c.A644G/G937T | N215S/D313Y |
| c.646T>G | c.T646G | Y216D |
| c.647A>C | c.A647C | Y216S |
| c.647A>G | c.A647G | Y216C |
| c.655A>C | c.A655C | I219L |
| c.656T>A | c.T656A | I219N |
| c.656T>C | c.T656C | I219T |
| c.659G>A | c.G659A | R220Q |
| c.659G>C | c.G659C | R220P |
| c.662A>C | c.A662C | Q221P |
| c.671A>C | c.A671C | N224T |
| c.671A>G | c.A671G | N224S |
| c.673C>G | c.C673G | H225D |
| c.683A>G | c.A683G | N228S |
| c.687T>A or c.687T>G | c.T687A or c.T687G | F229L |
| c.695T>C | c.T695C | I232T |
| c.713G>A | c.G713A | S238N |
| c.716T>C | c.T716C | I239T |
| c.720G>C or c.720G>T | c.G720C or c.G720T | K240N |
| c.724A>G | c.A724G | I242V |
| c.724A>T | c.A724T | I242F |
| c.725T>A | c.T725A | I242N |
| c.725T>C | c.T725C | I242T |
| c.728T>G | c.T728G | L243W |
| c.729G>C or c.729G>T | c.G729C or c.G729T | L243F |
| c.730G>A | c.G730A | D244N |
| c.730G>C | c.G730C | D244H |
| c.733T>G | c.T733G | W245G |
| c.740C>G | c.C740G | S247C |
| c.747C>G or c.747C>A | c.C747G or c.C747A | N249K |
| c.748C>A | c.C748A | Q250K |
| c.749A>C | c.A749C | Q250P |
| c.749A>G | c.A749G | Q250R |
| c.750G>C | c.G750C | Q250H |
| c.758T>C | c.T758C | I253T |
| c.758T>G | c.T758G | I253S |
| c.760-762delGTT | c.760_762delGTT | p.V254del |
| c.769G>C | c.G769C | A257P |
| c.770C>G | c.C770G | A257G |
| c.772G>C or c.772G>A | c.G772C or c.G772A | G258R |
| c.773G>T | c.G773T | G258V |
| c.776C>G | c.C776G | P259R |
| c.776C>T | c.C776T | P259L |
| c.779G>A | c.G779A | G260E |
| c.779G>C | c.G779C | G260A |
| c.781G>A | c.G781A | G261S |
| c.781G>C | c.G781C | G261R |
| c.781G>T | c.G781T | G261C |
| c.788A>G | c.A788G | N263S |
| c.790G>T | c.G790T | D264Y |
| c.794C>T | c.C794T | P265L |
| c.800T>C | c.T800C | M267T |
| c.805G>A | c.G805A | V269M |
| c.806T>C | c.T806C | V269A |
| c.809T>C | c.T809C | I270T |
| c.810T>G | c.T810G | I270M |
| c.811G>A | c.G811A | G271S |
| c.[811G>A; 937G>T] | c.G811A/G937T | G271S/D313Y |
| c.812G>A | c.G812A | G271D |
| c.823C>G | c.C823G | L275V |
| c.827G>A | c.G827A | S276N |
| c.829T>G | c.T829G | W277G |
| c.831G>T or c.831G>C | c.G831T or c.G831C | W277C |
| c.832A>T | c.A832T | N278Y |
| c.835C>G | c.C835G | Q279E |
| c.838C>A | c.C838A | Q280K |
| c.840A>T or c.840A>C | c.A840T or c.A840C | Q280H |
| c.844A>G | c.A844G | T282A |
| c.845C>T | c.C845T | T282I |
| c.850A>G | c.A850G | M284V |
| c.851T>C | c.T851C | M284T |
| c.860G>T | c.G860T | W287L |
| c.862G>C | c.G862C | A288P |
| c.866T>G | c.T866G | I289S |
| c.868A>C or c.868A>T | c.A868C or c.A868T | M290L |
| c.869T>C | c.T869C | M290T |
| c.870G>A or c.870G>C or c.870G>T | c.G870A or c.G870C or c.G870T | M290I |
| c.871G>A | c.G871A | A291T |
| c.877C>A | c.C877A | P293T |
| c.881T>C | c.T881C | L294S |
| c.884T>G | c.T884G | F295C |
| c.886A>G | c.A886G | M296V |
| c.886A>T or c.886A>C | c.A886T or c.A886C | M296L |
| c.887T>C | c.T887C | M296T |
| c.888G>A or c.888G>T or c.888G>C | c.G888A or c.G888T or c.G888C | M296I |
| c.893A>G | c.A893G | N298S |
| c.897C>G or c.897C>A | c.C897G or c.C897A | D299E |
| c.898C>T | c.C898T | L300F |
| c.899T>C | c.T899C | L300P |
| c.901C>G | c.C901G | R301G |
| c.902G>C | c.G902C | R301P |
| c.902G>A | c.G902A | R301Q |
| c.902G>T | c.G902T | R301L |
| c.907A>T | c.A907T | I303F |
| c.908T>A | c.T908A | I303N |
| c.911G>A | c.G911A | S304N |
| c.911G>C | c.G911C | S304T |
| c.919G>A | c.G919A | A307T |
| c.922A>G | c.A922G | K308E |
| c.924A>T or c.924A>C | c.A924T or c.A924C | K308N |
| c.925G>C | c.G925C | A309P |
| c.926C>T | c.C926T | A309V |
| c.928C>T | c.C928T | L310F |
| c.931C>G | c.C931G | L311V |
| c.935A>G | c.A935G | Q312R |
| c.936G>T or c.936G>C | c.G936T or c.G936C | Q312H |
| c.937G>T | c.G937T | D313Y |
| c.[937G>T; 1232G>A] | c.G937T/G1232A | D313Y/G411D |
| c.938A>G | c.A938G | D313G |
| c.946G>A | c.G946A | V316I |
| c.947T>G | c.T947G | V316G |
| c.950T>C | c.T950C | I317T |
| c.955A>T | c.A955T | I319F |
| c.956T>C | c.T956C | I319T |
| c.959A>T | c.A959T | N320I |
| c.962A>G | c.A962G | Q321R |
| c.962A>T | c.A962T | Q321L |
| c.963G>C or c.963G>T | c.G963C or c.G963T | Q321H |
| c.964G>A | c.G964A | D322N |
| c.964G>C | c.G964C | D322H |
| c.966C>A or c.966C>G | c.C966A or c.C966G | D322E |
| c.968C>G | c.C968G | P323R |
| c.973G>A | c.G973A | G325S |
| c.973G>C | c.G973C | G325R |
| c.978G>C or c.978G>T | c.G978C or c.G978T | K326N |
| c.979C>G | c.C979G | Q327E |
| c.980A>T | c.A980T | Q327L |
| c.983G>C | c.G983C | G328A |
| c.989A>G | c.A989G | Q330R |
| c.1001G>A | c.G1001A | G334E |
| c.1010T>C | c.T1010C | F337S |
| c.1012G>A | c.G1012A | E338K |
| c.1016T>A | c.T1016A | V339E |
| c.1027C>A | c.C1027A | P343T |
| c.1028C>T | c.C1028T | P343L |
| c.1033T>C | c.T1033C | S345P |
| c.1046G>C | c.G1046C | W349S |
| c.1055C>G | c.C1055G | A352G |
| c.1055C>T | c.C1055T | A352V |
| c.1061T>A | c.T1061A | I354K |
| c.1066C>G | c.C1066G | R356G |
| c.1066C>T | c.C1066T | R356W |
| c.1067G>A | c.G1067A | R356Q |
| c.1067G>C | c.G1067C | R356P |
| c.1072G>C | c.G1072C | E358Q |
| c.1073A>C | c.A1073C | E358A |
| c.1073A>G | c.A1073G | E358G |
| c.1074G>T or c.1074G>C | c.G1074T or c.G1074C | E358D |
| c.1076T>C | c.T1076C | I359T |
| c.1078G>A | c.G1078A | G360S |
| c.1078G>T | c.G1078T | G360C |
| c.1079G>A | c.G1079A | G360D |
| c.1082G>A | c.G1082A | G361E |
| c.1082G>C | c.G1082C | G361A |
| c.1084C>A | c.C1084A | P362T |
| c.1085C>T | c.C1085T | P362L |
| c.1087C>T | c.C1087T | R363C |
| c.1088G>A | c.G1088A | R363H |
| c.1102G>A | c.G1102A | A368T |
| c.1117G>A | c.G1117A | G373S |
| c.1124G>A | c.G1124A | G375E |
| c.1153A>G | c.A1153G | T385A |
| c.1168G>A | c.G1168A | V390M |
| c.1172A>C | c.A1172C | K391T |
| c.1175G>C | c.G1175C | R392T |
| c.1184G>A | c.G1184A | G395E |
| c.1184G>C | c.G1184C | G395A |
| c.1192G>A | c.G1192A | E398K |
| c.1202_1203insGACTTC | c.1202_1203insGACTTC | p.T400_S401dup |
| c.1208T>C | c.T1208C | L403S |
| c.1225C>G | c.C1225G | P409A |
| c.1225C>T | c.C1225T | P409S |
| c.1225C>A | c.C1225A | P409T |
| c.1228A>G | c.A1228G | T410A |
| c.1229C>T | c.C1229T | T410I |
| c.1232G>A | c.G1232A | G411D |
| c.1235C>A | c.C1235A | T412N |
| c.1253A>G | c.A1253G | E418G |
| c.1261A>G | c.A1261G | M421V |

### Dosing, Formulation and Administration

In one or more embodiments, the Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

The effective amount of migalastat or salt thereof can be in the range from about 100 mg FBE to about 150 mg FBE. Exemplary doses include about 100 mg FBE, about 105 mg FBE, about 110 mg FBE, about 115 mg FBE, about 120 mg FBE, about 123 mg FBE, about 125 mg FBE, about 130 mg FBE, about 135 mg FBE, about 140 mg FBE, about 145 mg FBE or about 150 mg FBE.

Again, it is noted that 150 mg of migalastat hydrochloride is equivalent to 123 mg of the free base form of migalastat. Thus, in one or more embodiments, the dose is 150 mg of migalastat hydrochloride or an equivalent dose of migalastat or a salt thereof other than the hydrochloride salt, administered at a frequency of once every other day. As set forth above, this dose is referred to as 123 mg FBE of migalastat. In further embodiments, the dose is 150 mg of migalastat hydrochloride administered at a frequency of once every other day. In other embodiments, the dose is 123 mg of the migalastat free base administered at a frequency of once every other day.

In various embodiments, the effective amount is about 122 mg, about 128 mg, about 134 mg, about 140 mg, about 146 mg, about 150 mg, about 152 mg, about 159 mg, about 165 mg, about 171 mg, about 177 mg or about 183 mg of migalastat hydrochloride.

Accordingly, in various embodiments, migalastat therapy includes administering 123 mg FBE at a frequency of once every other day, such as 150 mg of migalastat hydrochloride every other day.

The administration of migalastat or salt thereof may be for a certain period of time. In one or more embodiments, the migalastat or salt thereof is administered for a duration of at least 28 days, such as at least 30, 60 or 90 days or at least 3, 4, 5, 6, 7, 8, 9, 10, 11, 12, 16, 20, 24, 30 or 36 months or at least 1, 2, 3, 4 or 5 years. In various embodiments, the migalastat therapy is long-term migalastat therapy of at least 6 months, such as at least 6, 7, 8, 9, 10, 11, 12, 16, 20, 24, 30 or 36 months or at least 1, 2, 3, 4 or 5 years.

Administration of migalastat or salt thereof according to the present invention may be in a formulation suitable for any route of administration, but is preferably administered in an oral dosage form such as a tablet, capsule or solution. As one example, the patient is orally administered capsules each containing 150 mg migalastat hydrochloride or an equivalent dose of migalastat or a salt thereof other than the hydrochloride salt.

In some embodiments, the PC (e.g., migalastat or salt thereof) is administered orally. In one or more embodiments, the PC (e.g., migalastat or salt thereof) is administered by injection. The PC may be accompanied by a pharmaceutically acceptable carrier, which may depend on the method of administration.

In one or more embodiments, the PC (*e*.*g.,* migalastat or salt thereof) is administered as monotherapy, and can be in a form suitable for any route of administration, including *e.g*., orally in the form tablets or capsules or liquid, or in sterile aqueous solution for injection. In other embodiments, the PC is provided in a dry lyophilized powder to be added to the formulation of the replacement enzyme during or immediately after reconstitution to prevent enzyme aggregation *in vitro* prior to administration.

When the PC (*e.g.,* migalastat or salt thereof) is formulated for oral administration, the tablets or capsules can be prepared by conventional means with pharmaceutically acceptable excipients such as binding agents (*e.g*., pregelatinized maize starch, polyvinylpyrrolidone or hydroxypropyl methylcellulose); fillers (*e.g*., lactose, microcrystalline cellulose or calcium hydrogen phosphate); lubricants (*e.g.*, magnesium stearate, talc or silica); disintegrants (*e.g.*, potato starch or sodium starch glycolate); or wetting agents (*e.g.*, sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of the PC (*e.g.,* migalastat or salt thereof) suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and the PC (e.g., migalastat or salt thereof) in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, bicarbonate buffer, amino acids, urea, alcohols, ascorbic acid, and phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol *(e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external (*e.g.,* an i.v. bag) or internal *(e.g.,* a bioerodable implant).

Embodiments relating to pharmaceutical formulations and administration may be combined with any of the other embodiments of the invention, for example embodiments relating to methods of treating patients with Fabry disease, methods of treating ERT-naïve Fabry patients, methods of treating ERT-experienced Fabry patients, methods of enhancing cardiac function *(e.g.,* left ventricular systolic function), methods of stabilizing cardiac function *(e.g.,* left ventricular systolic function), methods of increasing MWFS, methods of stabilizing MWFS, methods of normalizing MWFS, methods of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, the PCs and suitable dosages thereof.

In one or more embodiments, the PC (*e.g.,* migalastat or salt thereof) is administered in combination with ERT. ERT increases the amount of protein by exogenously introducing wild-type or biologically functional enzyme by way of infusion. This therapy has been developed for many genetic disorders, including LSDs such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme (*e.g.,* replacement α-Gal A). In some embodiments, the chaperone is co-formulated with the replacement enzyme (*e.g.,* replacement α-Gal A).

In one or more embodiments, a patient is switched from ERT to migalastat therapy. In some embodiments, a patient on ERT is identified, the patient's ERT is discontinued, and the patient begins receiving migalastat therapy. The migalastat therapy can be in accordance with any of the methods described herein.

### Cardiac Function

The dosing regimens described herein can stabilize and/or enhance cardiac function *(e.g.,* left ventricular systolic function) in Fabry patients. As untreated Fabry patients typically exhibit a deterioration of cardiac function over time, both enhancements in and maintenance of cardiac function are indications of a benefit of migalastat therapy. As described in further detail in the Examples below, Phase 3 studies have found that migalastat therapy increases and/or stabilizes MWFS in both ERT-experienced and ERT-naïve patients. These Phase 3 studies also found that migalastat therapy normalizes MWFS in some patients with impaired MWFS. Accordingly, migalastat therapy can be used to treat Fabry patients by stabilizing MWFS, increasing MWFS and/or normalizing MWFS in ERT-naïve and/or ERT-experienced Fabry patients, including patients with impaired MWFS.

The migalastat therapy may arrest or decrease the reduction in MWFS and/or increase MWFS for a Fabry patient compared to the same patient without treatment with migalastat therapy. In one or more embodiments, the migalastat therapy provides a change in MWFS for a patient that is greater than (i.e., more positive than) -2%, such as greater than or equal to about -1.5%, -1.4%, -1.3%, -1.2%, -1.1%, -1%, -0.9%, -08.%, -0.7%, -0.6%, -0.5%, - 0.4%, -0.3%, -0.2%, -0.1%, 0%, 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.1%, 2.2%, 2.3%, 2.4% or 2.5%. In one or more embodiments, the Fabry patient is an ERT-experienced patient. In one or more embodiments, the Fabry patient is an ERT-naïve patient. In one or more embodiments, the Fabry patient has impaired MWFS prior to initiating migalastat therapy.

In one or more embodiments, the migalastat therapy provides an average change of MWFS in a group of ERT-naïve patients of at least about 0% after 12 months of administration of migalastat or a salt thereof. In various embodiments, the average increase in the group of ERT-naïve patients after 12 months of administration of migalastat or a salt thereof is at least about 0.05%, about 0.1%, about 0.15% or about 0.2%. In one or more embodiments, the ERT-naïve patients have impaired MWFS prior to initiating migalastat therapy.

In one or more embodiments, the migalastat therapy provides an average change of MWFS in a group of ERT-naïve patients of at least about 0% after 24 months of administration of migalastat or a salt thereof. In various embodiments, the average increase in the group of ERT-naïve patients after 12 months of administration of migalastat or a salt thereof is at least about 0.05%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4% or about 1.5%. In one or more embodiments, the ERT-naïve patients have impaired MWFS prior to initiating migalastat therapy.

In one or more embodiments, the migalastat therapy provides an average change of MWFS in a group of ERT-naïve patients of at least about 0% after 36 months of administration of migalastat or a salt thereof. In various embodiments, the average increase in the group of ERT-naïve patients after 12 months of administration of migalastat or a salt thereof is at least about 0.05%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4% or about 1.5%. In one or more embodiments, the ERT-naïve patients have impaired MWFS prior to initiating migalastat therapy.

In one or more embodiments, the migalastat therapy provides an average change of MWFS in a group of ERT-naïve patients of at least about 0% after 48 months of administration of migalastat or a salt thereof. In various embodiments, the average increase in the group of ERT-naïve patients after 12 months of administration of migalastat or a salt thereof is at least about 0.05%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9% about 2%, about 2.1%, about 2.2%, about 2.3%, about 2.4% or about 2.5%. In one or more embodiments, the ERT-naïve patients have impaired MWFS prior to initiating migalastat therapy.

In one or more embodiments, the migalastat therapy provides an average change of MWFS in a group of ERT-naïve patients of greater about -1.5% after 30 months of administration of migalastat or a salt thereof. In various embodiments, the average increase in the group of ERT-naïve patients after 12 months of administration of migalastat or a salt thereof is greater than -1.5%, -1.4%, -1.3%, -1.2%, -1.1%, -1%, -0.9%, -08.%, -0.7%, -0.6%, - 0.5%, -0.4%, -0.3%, -0.2%, -0.1% or 0%. In one or more embodiments, the ERT-experienced patients have impaired MWFS prior to initiating migalastat therapy.

### EXAMPLES

### EXAMPLE 1: Dosing Regimens for the Treatment of ERT-Naïve Fabry Patients Using Migalastat Hydrochloride

This example describes a Phase 3 study of migalastat therapy in ERT-naïve Fabry patients.

***Patient Enrollment.*** Eligible patients were 16-74 years old and had genetically-confirmed Fabry disease; had either never received or had not received ERT for ≥6 months; had a GLA mutation that resulted in a mutant protein that would respond to migalastat, based on the human embryonic kidney-293 (HEK) assay used at the time of enrollment; had an eGFR >30 ml/minute/1.73m², and had a urinary GL-3 ≥4 times the upper limit of normal.

***Study Design.*** Following eligibility-baseline assessments (2 months), patients were randomized to Stage 1-- 6 months of double-blind administration of 150 mg migalastat hydrochloride or placebo every other day. All patients completing Stage 1 were eligible to receive open-label migalastat in Stage 2 (months 6-12) and for an additional year (months 13-24) thereafter. The primary objective was to compare the effect of migalastat to placebo on kidney GL-3 as assessed by histological scoring of the number of inclusions in interstitial capillaries after 6 months of treatment. The secondary objectives of Stage 1 were to compare the effect of migalastat to placebo on urine GL-3 levels, on renal function, 24-hours urinary protein, and on safety and tolerability. The tertiary objectives were cardiac function, patient-reported outcomes, exploratory kidney analyses, and white blood cell α-Gal A activity. Study completers were eligible to enroll in the open-label extension study for up to 5 years.

***Kidney Histology Assessment.*** Each patient underwent a baseline kidney biopsy, as well as repeat kidney biopsies at 6 and 12 months. The number of GL-3 inclusions per kidney interstitial capillary per patient at baseline, and at 6 and 12 months was quantitatively assessed in 300 capillaries by 3 independent pathologists blinded to treatment and visit. All values for each individual biopsy at a given time were averaged prior to statistical analysis.

GL-3 changes in podocytes, endothelial cells, and mesangial cells, and glomerular sclerosis, were assessed qualitatively by the same 3 pathologists blinded to treatment/visit.

***Globotriaosylceramide and Globotriaosylsphingosine.*** Plasma lyso-Gb3 and 24-hour urine GL-3 were analyzed by liquid chromatography-mass-spectroscopy using a novel stable isotope-labeled internal standard, 13C6-lyso-Gb3 (lower-limit-of-quantification: 0.200 ng/mL, 0.254 nmol/L).

***Renal Function Assessment.*** Annualized rates of change (mL/min/1.73m²/year) were calculated using Chronic Kidney Disease Epidemiology Collaboration-eGFR_{CKD-EPI}) and measured iohexol clearance-mGFRᵢₒₕₑₓₒₗ).

***Echocardiography.*** LVMi, left posterior wall thickness, diastolic, interventricular septum thickness, diastolic and other parameters were assessed through blinded, centralized evaluation.

***Patient-Reported Outcomes.*** Patient-reported outcomes were assessed using the Gastrointestinal-Symptoms-Rating-Scale (GSRS), Short Form-36v2TM and Brief-Pain-Inventory-Pain-Severity-Component.

***Safety Analysis and Adverse Events.*** Randomized patients receiving ≥1 dose were included in the safety analysis, which comprised vital signs, physical exams, electrocardiograms, clinical labs, and adverse events.

***Statistical Analyses for Kidney Interstitial Capillary GL-3 Substrate.*** The primary Stage 1 (6 month) endpoint (ITT population with baseline biopsies, n=64) was the proportion of patients in the migalastat and placebo groups with a ≥50% reduction in GL-3 inclusions per interstitial capillary. Two other Stage 1 endpoints were assessed (modified-ITT population: randomized patients with paired baseline and month 6 biopsies; n=60): percent change in GL-3 inclusions per interstitial capillary, and percent of interstitial capillaries with zero GL-3 inclusions.

Efficacy analyses for GL-3 inclusions per interstitial capillary and other prespecified endpoints in Stage 2 (months 6-12) and the open-label-extension (months 12-24) were based on the modified intention to treat (mITT) - population consisting of randomized patients with mutant α-Gal A enzyme shown to be suitable for migalastat treatment by the validated assay; n=50).

### Results

**Baseline Characteristics.** Sixty-seven patients (16-74 years-old; 64% female) with potentially responsive mutant α-Gal A were randomized (ITT population). Table 2 provides the baseline characteristics for the 50 patients in the ITT population with suitable mutant α-Gal A. There were no statistically significant differences in baseline parameters.

**Table 2: Baseline Characteristics**

| **Parameter** | | **Treatment Group** | | **Total (N=50)** |
|---|---|---|---|---|
| | | **Migalastat HCl** (N=28) | **Placebo to Migalastat HCl** (N=22) | |
| **Age (year) (n)** | | 28 | 22 | 50 |
| | Mean±SD | 41.5±13 | 45.1±8.0 | 43.1±11 |
| | Median | 37.0 | 45.5 | 45.0 |
| **Weight (kg) (n)** | | 28 | 22 | 50 |
| | Mean±SD | 72.6±15.35 | 76.1±16.52 | 74.1±15.81 |
| | Median | 72.3 | 74.0 | 72.8 |
| | **Number of Years of Diagnosis of Fabrv Disease (n)** | 28 | 21 | 49 |
| | Mean±SD | 5.6±6.89 | 7.3±8.80 | 6.3±7.73 |
| | Median | 4.1 | 4.1 | 4.1 |
| **Number of patients previously on ERT (>6 months prior to baseline) (%)** | | 4 (14.3%) | 7 (31.8%) | 11 (22.0%) |

| Use of ACEi/ARB/Ri at Baseline | | | | |
|---|---|---|---|---|
| | Yes (%) | 9 (32.1%) | 12 (54.5%) | 21 (42.0%) |
| | No (%) | 19 (67.9%) | 10 (45.5%) | 29 (58.0%) |
| **Proteinuria >150mg/24h (%)** | | 17 (60.7%) | 18 (81.8%) | 35 (70.0%) |
| **Proteinuria >300mg/24h (%)** | | 8 (28.6%) | 11 (50.0%) | 19 (38.0%) |
| **Proteinuria >1000mg/24h (%)** | | 3 (10.7%) | 3 (13.6%) | 6 (12.0%) |
| **mGFR _{Iohexol} (mL/min/1.73m²) (n)** | | 27 | 21 | 48 |
| | Mean±SD | 79.95±30.9 | 83.12±22.8 | 81.34±27.5 |
| | Median | 84.90 | 82.20 | 83.40 |
| **eGFR_{CKD-EPI} (mL/min/1.73m²)** | | 28 | 22 | 50 |
| | Mean±SD | 94.4±27.0 | 90.6±17.1 | 92.7±23.0 |
| | Median | 96.6 | 93.5 | 94.0 |
| **Lyso-Gb₃ (n)** | | 18 | 13 | 31 |
| | Mean (nmol/L) ±SD | 47.3±62 | 41.9±39 | 45.0±53 |

Published reports of clinical phenotype(s) associated with the genotypes of patients with suitable mutations (n=50) indicate that 30 (60%) had mutations associated with the classic phenotype of Fabry disease, one (2%) with the non-classic phenotype, three (6%) with both phenotypes, and 16 (32%) not yet classified. Residual WBC α-Gal A activity <3% was found in 14 of 16 (87%) males; 29 of 31 (94%) males and females had elevated plasma lyso-Gb3, and 47 of 50 (94%) males and females had multi-organ system disease.

***Baseline MWFS.*** At baseline, impaired MWFS (<15% for females and <14% for males) was reported in 9 patients.

**Migalastat and Cardiac Function.** This study of ERT-naïve patients found that migalastat therapy increased MWFS in patients with impaired MWFS at baseline. Table 3 below shows the change from baseline in MWFS after migalastat therapy.

**Table 3: Percent Changes From Baseline in MWFS Over Time with Migalastat Therapy in Patients With Impaired MWFS at Baseline (Patients with Amenable Mutations)**

| | **Baseline Mean MWFS = 11.3 %** | | | | |
|---|---|---|---|---|---|
| | **Timepoint** | | | | |
| | **Month 12** | **Month 24** | **Month 36** | **Month 48** | **LOCF** |
| **n** | 7 | 8 | 4 | 3 | 8 |
| **Mean change from baseline** | 0.1% | 1.4% | 1.4% | 2.4% | 1.9% |
| **95% CI** | -1.2%, -1.4% | -1.3%, 4.0% | -1.5%, 4.3% | -2.1%, 6.9% | -0.8%, 4.5% |
| **Any increase** | 2/7 (29%) | 5/8 (63%) | 3/4 (75%) | *3*/*3* (100%) | 6/8 (75%) |
| **Normalization** | 0 | 2/8 (25%) | 2/4 (50%) | 2/3 (67%) | 3/8 (38%) |

| | | | | | |
|---|---|---|---|---|---|
| Last observation carried forward (LOCF) analyses are based on last study assessment including any unscheduled or early termination visits; Abnormal MWFS is <15% for females and <14% for males. | | | | | |

As can be seen from Table 3, LOCF analysis of ERT-naïve patients with impaired MWFS at baseline showed mean changes in MWFS of 1.9% (95% CI -0.8%, 4.5%; n=8) over 48 months of migalastat therapy. 6/8 (75%) of patients had an increase of MWFS after migalastat therapy, with 3/8 (38%) demonstrating normalization of MWFS.

MWFS was also analyzed in patients with LVH at baseline. The change from baseline in MWFS in patients with LVH at baseline is provided in Table 4 below:

**Table 4: Change From Baseline in MWFS With Migalastat in Patients With LVH at Baseline (Patients with Amenable Mutations)**

| | **Baseline** | **Change from Baseline** | | | | |
|---|---|---|---|---|---|---|
| | | **Month 12** | **Month 24** | **Month 36** | **Month 48** | **LOCF** |
| **n** | 10 | 9 | 9 | 5 | 4 | 10 |
| **%, mean (SD) or (95% CI)** | 12.2 (2.6) | 0.2 (-0.8, 1.1) | 0.9 (-1.6, 3.4) | 0.7 (-2.1, 3.4) | 0.6 (-5.7, 6.9) | 1.0 (-1.5, 3.5) |
| **Any increase** | - | 4/9 (44%) | 5/9 (56%) | 3/5 (60%) | 3/4 (75%) | 7/10 (70%) |
| **Normalization** | - | 2/9 (22%) | 2/9 (22%) | 1/5 (20%) | 0 | 2/10 (20%) |

| | | | | | | |
|---|---|---|---|---|---|---|
| LOCF analyses are based on last study assessment including any unscheduled or early termination visits; LVH subgroup: LVMi >95 g/m2 (females) or >115 g/m2 (males). | | | | | | |

As can be seen from Table 4, LOCF analysis of ERT-naïve patients with LVH at baseline showed mean changes in MWFS of 1.0% (95% CI -1.5%, 3.5%; n=10) over 48 months of migalastat therapy. 7/10 (70%) of patients had an increase of MWFS after migalastat therapy, with 2/10 (20%) demonstrating normalization of MWFS.

**Safety and Adverse Events.** During Stage 1, the treatment-emergent adverse events were similar between groups. Adverse events with a higher frequency in patients receiving migalastat compared to placebo were headache (12/34 patients-35% versus 7/33 patients-21%) and nasopharyngitis (6/34 patients-18% versus 2/34-6%). The most frequently reported adverse events for Stage 2 were headache (9/63 patients-14%) and procedural pain (7/63 patients-11%-related to kidney biopsies) and, for the open-label-extension, proteinuria (9/57 patients-16%), headache (6/57 patients-11%), and bronchitis (6/57 patients-11%). Most adverse events were mild or moderate in severity. No adverse events led to migalastat discontinuation.

Six patients experienced serious adverse events during Stage 1 (2: migalastat; 4: placebo), 5 during Stage 2, and 11 during the open-label-extension. Two serious adverse events were assessed as possibly related to migalastat by the investigator-- fatigue and paresthesia. Both occurred in the same patient between months 12-24 and resolved. No individual serious adverse event was reported by >1 patient. Two patients discontinued migalastat due to serious adverse events; both were deemed unrelated to migalastat. No deaths were reported.

Treatment-emergent proteinuria was reported in 9 patients (16%) between months 12-24, and in one case, was judged as migalastat-related. In 5 patients, the 24-month values were in the same range as baseline. Three patients with suitable mutations had overt baseline proteinuria (>1g/24-hr), which increased over 24 months. In 23/28 patients with baseline proteinuria <300 mg/24-h, 24-hour urine protein remained stable during migalastat treatment.

There was no progression to end-stage renal disease, no cardiac death and no stroke as defined in Banikazemi et al.. There was a single case of transient ischemic attack-judged unrelated to migalastat.

Analyses of vital sign, physical findings, laboratory, and ECG parameters did not reveal any clinically relevant effect of migalastat.

### EXAMPLE 2: Dosing Regimens for the Treatment of ERT-Experienced Fabry Patients Using Migalastat Hydrochloride

This example describes a Phase 3 study of migalastat therapy in ERT-experienced Fabry patients.

***Patient Enrollment.*** Eligible patients were 16-74 years old and had genetically-confirmed Fabry disease; had received ERT for ≥12 months; had a GLA mutation that resulted in a mutant protein that would respond to migalastat, based on the human embryonic kidney-293 (HEK) assay used at the time of enrollment; had an eGFR ≥ 30 ml/minute/1.73m²; and had an ERT dose level and regimen that had been stable for at least 3 months.

***Study Design.*** Following eligibility-baseline assessments, 57 patients were randomized to 18 months of migalastat therapy or ERT, followed by followed by 12 months of migalastat therapy. The migalastat dosing regimen was 150 mg of migalastat hydrochloride every other day. The primary objective was to compare the effect of migalastat to ERT on renal function assessed by mGFRᵢₒₕₑₓₒₗ after 18 months of treatment. The secondary objectives were to compare the effect of migalastat to ERT on: renal function (assessed by eGFR and 24-hour urine protein); composite clinical outcome (assessed by time to occurrence of renal, cardiac, cerebrovascular events or death); cardiac function (assessed by echocardiography) and patient reported outcomes (pain and quality of life).

***Baseline MWFS.*** At baseline, impaired MWFS (<15% for females and <14% for males) was reported in 19 (14 migalastat, 5 ERT) patients.

### Results

**Migalastat and Cardiac Function.** This study of ERT-experienced patients found that migalastat therapy stabilized MWFS in patients with impaired MWFS at baseline. LOCF analysis of patients with impaired MWFS at baseline showed mean changes in baseline of -0.2% (95% CI -1.3%, 1.0%; n=14) over 30 months of migalastat therapy. LOCF analysis of patients with impaired MWFS at baseline showed mean changes in MWFS of -0.6% (95% CI -2.6%, 1.4%; n=5) over 18 months of treatment with ERT.

The embodiments described herein are intended to be illustrative of the present compositions and methods and are not intended to limit the scope of the present invention. Various modifications and changes consistent with the description as a whole and which are readily apparent to the person of skill in the art are intended to be included. The appended claims should not be limited by the specific embodiments set forth in the examples, but should be given the broadest interpretation consistent with the description as a whole.

Patents, patent applications, publications, product descriptions, GenBank Accession Numbers, and protocols are cited throughout this application, the disclosures of which are incorporated herein by reference in their entireties for all purposes.

The present invention is also exemplified by the following preferred embodiments:
1. A method of enhancing cardiac function in a patient having Fabry disease, the method comprising administering to the patient a formulation comprising an effective amount of migalastat or salt thereof every other day for enhancing the patient's cardiac function, wherein the effective amount is about 100 mg to about 150 mg free base equivalent (FBE).
2. The method of item 1, wherein enhancing cardiac function comprises enhancing left ventricular systolic function.
3. The method of item 1 or 2, wherein the patient has impaired midwall fractional shortening (MWFS) prior to initiating administration of the migalastat or salt thereof.
4. The method of any one of items 1-3, wherein the migalastat or salt thereof enhances α-galactosidase A activity.
5. The method of any one of items 1-4, wherein the patient is administered about 123 mg FBE of the migalastat or salt thereof every other day.
6. The method of any one of items 1-5, wherein the patient is administered about 123 mg of migalastat free base every other day.
7. The method of any one of items 1-5, wherein the patient is administered about 150 mg of migalastat hydrochloride every other day.
8. The method of any one of items 1-7, wherein the formulation comprises an oral dosage form.
9. The method of item 8, wherein the oral dosage form comprises a tablet, a capsule or a solution.
10. The method of any one of items 1-9, wherein the migalastat or salt thereof is administered for at least 12 months.
11. The method of any one of items 1-10, wherein the migalastat or salt thereof is administered for at least 24 months.
12. The method of any one of items 1-11, wherein the patient is an enzyme replacement therapy (ERT)-naïve patient.
13. The method of any one of items 1-12, wherein the administration of migalastat or a salt thereof provides an average increase in MWFS in a group of ERT-naïve patients with impaired MWFS of at least about 1% after 24 months of administration of migalastat or a salt thereof.
14. The method of any one of items 1-11, wherein the patient is an ERT-experienced patient.
15. The method of any one of items 1-14, wherein the patient has a HEK assay amenable mutation in α-galactosidase A.
16. The method of item 15, wherein the mutation is disclosed in a pharmacological reference table.
17. The method of item 16, wherein the pharmacological reference table is provided in a product label for a migalastat product approved for the treatment of Fabry disease.
18. The method of item 16, wherein the pharmacological reference table is provided in a product label for GALAFOLD^{®}.
19. The method of item 16, wherein the pharmacological reference table is provided at a website.
20. The method of item 19, wherein the website is one or more of www.galafoldamenabilitytable.com or www.fabrygenevariantsearch.com.
21. A method of increasing midwall fractional shortening (MWFS) in a patient having Fabry disease, the method comprising administering to the patient a formulation comprising an effective amount of migalastat or salt thereof every other day for increasing the patient's MWFS, wherein the effective amount is about 100 mg to about 150 mg free base equivalent (FBE).
22. The method of item 21, wherein the patient has impaired MWFS prior to initiating administration of the migalastat or salt thereof.
23. The method of item 21or 22, wherein the migalastat or salt thereof enhances α-galactosidase A activity.
24. The method of any one of items 21-23, wherein the patient is administered about 123 mg FBE of the migalastat or salt thereof every other day.
25. The method of any one of items 21-24, wherein the patient is administered about 123 mg of migalastat free base every other day.
26. The method of any one of items 21-24, wherein the patient is administered about 150 mg of migalastat hydrochloride every other day.
27. The method of any one of items 21-26, wherein the formulation comprises an oral dosage form.
28. The method of item 27, wherein the oral dosage form comprises a tablet, a capsule or a solution.
29. The method of any one of items 21-28, wherein the migalastat or salt thereof is administered for at least 12 months.
30. The method of any one of items 21-29, wherein the migalastat or salt thereof is administered for at least 24 months.
31. The method of any one of items 21-30, wherein the patient is an enzyme replacement therapy (ERT)-naïve patient.
32. The method of any one of items 21-31 wherein the administration of migalastat or a salt thereof provides an average increase in MWFS in a group of ERT-naïve patients with impaired MWFS of at least about 1% after 24 months of administration of migalastat or a salt thereof.
33. The method of any one of items 21-30, wherein the patient is an ERT-experienced patient.
34. The method of any one of items 21-33, wherein the patient has a HEK assay amenable mutation in α-galactosidase A.
35. The method of item 34, wherein the mutation is disclosed in a pharmacological reference table.
36. The method of item 35, wherein the pharmacological reference table is provided in a product label for a migalastat product approved for the treatment of Fabry disease.
37. The method of item 35, wherein the pharmacological reference table is provided in a product label for GALAFOLD^{®}.
38. The method of item 35, wherein the pharmacological reference table is provided at a website.
39. The method of item 38, wherein the website is one or more of www.galafoldamenabilitytable.com or www.fabrygenevariantsearch.com.
40. A method of normalizing midwall fractional shortening (MWFS) in a patient having Fabry disease and impaired MWFS, the method comprising administering to the patient a formulation comprising an effective amount of migalastat or salt thereof every other day for normalizing the patient's MWFS, wherein the effective amount is about 100 mg to about 150 mg free base equivalent (FBE).
41. The method of item 40, wherein the migalastat or salt thereof enhances α-galactosidase A activity.
42. The method of item 40 or 41, wherein the patient is administered about 123 mg FBE of the migalastat or salt thereof every other day.
43. The method of any one of items 40-42, wherein the patient is administered about 123 mg of migalastat free base every other day.
44. The method of any one of items 40-42, wherein the patient is administered about 150 mg of migalastat hydrochloride every other day.
45. The method of any one of items 40-44, wherein the formulation comprises an oral dosage form.
46. The method of item 45, wherein the oral dosage form comprises a tablet, a capsule or a solution.
47. The method of any one of items 40-46, wherein the migalastat or salt thereof is administered for at least 12 months.
48. The method of any one of items 40-47, wherein the migalastat or salt thereof is administered for at least 24 months.
49. The method of any one of items 40-48, wherein the patient is an enzyme replacement therapy) ERT-naïve patient.
50. The method of any one of items 40-49, wherein the administration of migalastat or a salt thereof provides an average increase in MWFS in a group of ERT-naïve patients with impaired MWFS of at least about 1% after 24 months of administration of migalastat or a salt thereof.
51. The method of any one of items 40-50, wherein the patient has a HEK assay amenable mutation in α-galactosidase A.
52. The method of item 51, wherein the mutation is disclosed in a pharmacological reference table.
53. The method of item 52, wherein the pharmacological reference table is provided in a product label for a migalastat product approved for the treatment of Fabry disease.
54. The method of item 52, wherein the pharmacological reference table is provided in a product label for GALAFOLD^{®}.
55. The method of item 52, wherein the pharmacological reference table is provided at a website.
56. The method of item 55, wherein the website is one or more of www.galafoldamenabilitytable.com or www.fabrygenevariantsearch.com.
57. A method of stabilizing midwall fractional shortening (MWFS) in an enzyme replacement therapy (ERT)-experienced patient having Fabry disease, the method comprising administering to the patient a formulation comprising an effective amount of migalastat or salt thereof every other day for stabilizing the patient's MWFS, wherein the effective amount is about 100 mg to about 150 mg free base equivalent (FBE).
58. The method of item 57, wherein the patient has impaired MWFS prior to initiating administration of the migalastat or salt thereof.
59. The method of item 57 or 58, wherein the migalastat or salt thereof enhances α-galactosidase A activity.
60. The method of any one of items 57-59, wherein the patient is administered about 123 mg FBE of the migalastat or salt thereof every other day.
61. The method of any one of items 57-60, wherein the patient is administered about 123 mg of migalastat free base every other day.
62. The method of any one of items 57-60, wherein the patient is administered about 150 mg of migalastat hydrochloride every other day.
63. The method of any one of items 57-62, wherein the formulation comprises an oral dosage form.
64. The method of item 63, wherein the oral dosage form comprises a tablet, a capsule or a solution.
65. The method of any one of items 57-64, wherein the migalastat or salt thereof is administered for at least 12 months.
66. The method of any one of items 57-65, wherein the migalastat or salt thereof is administered for at least 30 months.
67. The method of any one of items 57-66, wherein the patient is an enzyme replacement therapy (ERT)-experienced patient.
68. The method of any one of items 57-67, wherein the administration of migalastat or a salt thereof provides an average change in MWFS in a group of ERT-experienced patients with impaired MWFS of greater than about -0.5% after 30 months of administration of migalastat or a salt thereof.
69. The method of any one of items 57-68, wherein the patient has a HEK assay amenable mutation in α-galactosidase A.
70. The method of item 69, wherein the mutation is disclosed in a pharmacological reference table.
71. The method of item 70, wherein the pharmacological reference table is provided in a product label for a migalastat product approved for the treatment of Fabry disease.
72. The method of item 70, wherein the pharmacological reference table is provided in a product label for GALAFOLD^{®}.
73. The method of item 70, wherein the pharmacological reference table is provided at a website.
74. The method of item 73, wherein the website is one or more of www.galafoldamenabilitytable.com or www.fabrygenevariantsearch.com.

## Claims

1. Migalastat or salt thereof for use in enhancing and/or stabilizing cardiac function in a patient having Fabry disease, wherein the patient is administered 123 mg free base equivalent (FBE) of the migalastat or salt thereof every other day, and wherein the patient has impaired midwall fractional shortening (MWFS) prior to initiating administration of the migalastat or salt thereof.

2. Migalastat or salt thereof for use according to claim 1, wherein the migalastat or salt thereof is comprised in a formulation, and wherein the formulation is administered to the patient.

3. Migalastat or salt thereof for use according to claim 1 or 2, wherein the patient is an enzyme replacement therapy (ERT)-naïve patient.

4. Migalastat or salt thereof for use according to any one of claims 1 to 3, wherein the administration of migalastat or a salt thereof provides an average increase in MWFS in a group of ERT-naïve patients with impaired MWFS of at least about 1% after 24 months of administration of migalastat or a salt thereof.

5. Migalastat or salt thereof for use according to claim 1 or 2, wherein the patient is an enzyme replacement therapy (ERT)-experienced patient.

6. Migalastat or salt thereof for use according to any one of claims 1-5, wherein the migalastat or salt thereof enhances α-galactosidase A activity.

7. Migalastat or salt thereof for use according to any one of claims 1-6, wherein the patient is administered 123 mg of migalastat free base every other day.

8. Migalastat or salt thereof for use according to any one of claims 1-6, wherein the patient is administered 150 mg of migalastat hydrochloride every other day.

9. Migalastat or salt thereof for use according to any one of claims 2-8, wherein the formulation comprises an oral dosage form.

10. Migalastat or salt thereof for use according to claim 9, wherein the oral dosage form comprises a tablet, a capsule or a solution.

11. Migalastat or salt thereof for use according to any one of claims 1-10, wherein the migalastat or salt thereof is administered for at least 12 months.

12. Migalastat or salt thereof for use according to any one of claims 1-10, wherein the migalastat or salt thereof is administered for at least 24 months.

13. Migalastat or salt thereof for use according to any one of claims 1-12, wherein the patient has a HEK assay amenable mutation in α-galactosidase A.


SEQUENCE LISTING

<110>  Amicus Therapeutics, Inc.

<120>  METHODS OF ENHANCING AND/OR STABILIZING CARDIAC FUNCTION IN
       PATIENTS WITH FABRY DISEASE

<130>  AT17-007-PCT

<150>  US 62/550,984
<151>  2017-08-28

<160>  3

<170>  PatentIn version 3.5

<210>  1
<211>  12436
<212>  DNA
<213>  Homo sapiens

<400>  1
cccttctgta ggggcagaga ggttctactt cattactgcg tctcctggga aggccatcag
60

gactgctggc taaagtggga accaggactc tttgtgagtt aagaatttgt gtatttatat
120

gtgtgttata cacatttttt aaaaaactgt aacgacatca ggttgagcag tcgtctccgg
180

gtggtgaatt atgtgtattt ttaaatttta tactatattg ttatttttca aatgttcgaa
240

attgaatatg tagattgttg ttatcagcag aaaaataaac attattcaaa tactctattc
300

agtaaagtaa tttattgggc gcctttgtca agcacgcatt tgcctagatg tgactctaca
360

gataaaattc acttggggcc tccccttaca gacaatcagg cagtggagac tgagtgcctg
420

aatggataga ccagcactca gaccactatt ttcagtatct gtttttctta actcagggcc
480

gtggttttca aacgtttttc gccttacggt cacccttagg gtcccccgag accggcccag
540

acagacagat atacaaaaac acatacacag tcatgagcgt ccaccatttc cccaccaggc
600

gcagcacagg cggcttcccg gcactgagat gggggggagg agggagagag cgcgaggggg
660

gaggggaaag cagagaacga aagaggcgga ggcggccccc gaaccccgct ctggtcttca
720

tcatcaccac ccctgggtcc ccagttccca cccacacacc aacctctaac gataccgggt
780

aattttcctc cttcttccct caaacggcta tagcgagacg gtagacgacg accagaacta
840

cttctgctca cgtaagcgag taatcacgtg agcgcctacg tcatgtgaga tctcggtcac
900

gtgagcaact ctcggcttaa actcgggatc actaaggtgc cgcacttcct tctggtatgg
960

aaatagggcg ggtcaatatc aagaaaggaa gagggtgatt ggttagcgga acgtcttacg
1020

tgactgatta ttggtctacc tctggggata accgtcccag ttgccagaga aacaataacg
1080

tcattattta ataagtcatc ggtgattggt ccgcccctga ggttaatctt aaaagcccag
1140

gttacccgcg gaaatttatg ctgtccggtc accgtgacaa tgcagctgag gaacccagaa
1200

ctacatctgg gctgcgcgct tgcgcttcgc ttcctggccc tcgtttcctg ggacatccct
1260

ggggctagag cactggacaa tggattggca aggacgccta ccatgggctg gctgcactgg
1320

gagcgcttca tgtgcaacct tgactgccag gaagagccag attcctgcat caggtatcag
1380

atattgggta ctcccttccc tttgcttttc catgtgtttg ggtgtgtttg gggaactgga
1440

gagtctcaac gggaacagtt gagcccgagg gagagctccc ccacccgact ctgctgctgc
1500

ttttttatcc ccagcaaact gtcccgaatc aggactagcc ctaaactttc tctgtgtgac
1560

ctttcctggg atgggagtcc ggccagcggc ccctgtttct ttctctctct ctctctctct
1620

cgttctcctt ctctttctct ttctcttctt tcctctctct ttctctctct ccctgcccgg
1680

ttctcttttt tcactgctcc ttgcagagca gggccacccc ataggcagtg tgcccaaagt
1740

agccctgccc ggttctattc agacccttct tgtgaacttc tgctcttcct ctgccgggtg
1800

ctaaccgtta gaacatctag ggtgggtagg aggaatgggg aactaagatt cgtgccattt
1860

tttctccttt tggggtcgtg gatttctcgg cagtatctcg agggagttag agagaccata
1920

aggtcgctga gatctctccc acctcgccca tgagcgtggc atcaggctgg aaggttgaca
1980

tggaggaact ttatacattt acacctttgc gtgagggttg aggctggatt agataggtat
2040

tgaacatatc tgaccctcac aatccttatc tgtaaattgg gattacaacc ttttaatttc
2100

agggagctga caaaaaaaat ctgaaaaata gttcttatct cacacaggtg agttttcaag
2160

gagataacct atttaaagta catagcacag cgcttgacca ttcaactgcg cttacagagc
2220

aaatgttcaa tgggaaaatg aatgtaaatc tacaaatctg aatgaatatg tgtatttttc
2280

tggagagagg atatttacct ttcttcaaat tctcaaaggg ctctgtgatt taaaaaaggt
2340

taggaatcac tgatagatgt tggtaaaagg tggcagtcac agtacatttc tgtgtccata
2400

agttattcct atgaatatct ttatagataa agtcaggatg ttggtcagac atcacagaag
2460

aaattggcct tgtaagtttc atgtgaccct gtggtacagt atgtgtggca attttgccca
2520

tcacggattt ttttttattg gtatttgcat ctgattataa aactaatgca tgatcattgc
2580

aaaaaatgta gataaagaag agcaaaatga aaataaagat ttccccccac cgttccacca
2640

cccagaaata atcatggttt aaatgttaat atacaacctt acaattgttt tctatataaa
2700

tgaaaacata gatttcttta tttcattatt ttccataaaa aatggatcat gtttatgtca
2760

tgtttggcta atggcaagac cctggcaccc agtctgggct caaattctgc ctcattgtta
2820

cttagccctg tgacattggg taaattacac tttttttttt tttttttttt tgagacgggg
2880

tctcgctctg tcgcccaggc tggagtgcag tggcacgatc tcggctcact gcaagtccgc
2940

ctcctgggtt cacgccattc ttctgcctca gcctcccgag tagctgggac tacaggcgcc
3000

tgccaccacg cctggctctt tttttttttt tttttttttt tagtacagac ggggtttcac
3060

catgttagcc agggtggtct caatctcctg acctcgtgat tcgcccgcct cagcctccca
3120

aagtgctggt gtgagccacc gtgcccagcc ttactttttt ttttgagagg gggtctcact
3180

ctgtcaccca ggttggagtg cagtggcgcg atctctgctc agtgcaaact ccacctcccg
3240

ggtttaagca gttctcctgt cgtagtctcc tgagtagctg ggattacagg cacaccacca
3300

cggccagcta atttttgtat tttcagtaga gacgggtttc accatgttgc ccaagctggt
3360

ctcgaactcc tggcctcaag tgatctgccc gccttggcct cccagagtgc tgggattaca
3420

ggtgtgagcc accgcacccg gcctcttttt tcttttttag tctatcatac cttgcaaata
3480

cagtggttct tcctatgtgt tggttttgat atttatgtaa tcaaacacat cagtttttcc
3540

tttctgattt ctgactttgg ggtcatgctg agaaagtcct ttcctacctg aagataatac
3600

agtatatacg tttcttacta gtatttttgt ggatttttaa aatatttaaa tctttagtcc
3660

atctgaactt gttcttctat cagaaatgcc acatttaata aataataagt cccatggtat
3720

cagatggctg gaaggacctc tttcgaaact ttgtttaatt ccattaatct gtgtattctt
3780

attctaatgc taatagttcc acactagctt cctttatctt ttttttcttt tttttttttt
3840

ttttgagctg gagtttcgct cttgttgccc aggctggagt acaatgtcac gatctcggtt
3900

caccgcaacc tccgcctccc aggttcaagc aattctcctg cctcatcctc gcgagtagct
3960

ggaattacag gcatgcgcca ccacgcctag ctattttgta tttttagtag agatggggtt
4020

tctccatgtt ggtcaggctg gtctcaaact cccagcctca ggtgatctgc ctgcctcggc
4080

ctcccaaaat gctgttatta caggcgtgag ccaccacgcc cagccttcat cttttaatga
4140

atgtacatgt atgtaatctt ttaggtgaac tttttgtaat gttgtgccaa gttccttaaa
4200

aagccctttt ggaagctggg caggtggcca cgcctgtaat cccagcattt tgggagtctg
4260

aggcaggtgg atcacttgag gccaggagtt caagactagc ctagccaaaa tgcaaaaccc
4320

tgtctctact aaagatacaa aaattagccg gatgcgatgg cacatgcctg taatctcagc
4380

tactcgggag gctgaggtag aagaatcgct tgaaccgggg aggcagaggt tgcagtgagc
4440

aagatggcgc cactgcactc cagcctgggt gacagaggga gactccatct caaaaaaaaa
4500

aaaaaaaaaa aagataaaaa ggaaacctaa gtactcttgg gctttgttaa ggattttgtt
4560

aaatatacaa aggattgcag ggaaaattaa cttattttta atattgagta tgcttatcca
4620

agagcaaaat aatatttctc catttattca aatcatttag gagcatcata gttttaacat
4680

atgggccttg cacgtatctt aaatttatct ctaggcattt taggttgttc agttgttctt
4740

gtgaatggga tctttttctc caaataggat tattgttgat atctgttgat tatgttaact
4800

ttgtagtttc tgactttact gaactgtctt cttagatcta atactctttt caatttcatc
4860

atatatttct cattcctatt ttgtttgggg tttttagggc gggaatatta acgggataag
4920

agagacaaaa gaaaatctgg aaaaacaatt cattttacct tacattgctt gtgattacta
4980

ccacactatt actgggttgg aaaaaattgt gaaatcccaa ggtgcctaat aaatgggagg
5040

tacctaagtg ttcatttaat gaattgtaat gattattgga atttctcttt cagtgagaag
5100

ctcttcatgg agatggcaga gctcatggtc tcagaaggct ggaaggatgc aggttatgag
5160

tacctctgca ttgatgactg ttggatggct ccccaaagag attcagaagg cagacttcag
5220

gcagaccctc agcgctttcc tcatgggatt cgccagctag ctaattatgt gagtttatag
5280

ataatgttct tgttcattca gaggactgta agcacttctg tacagaagct tgtttagaaa
5340

cagccctcat ggccgggcgt ggtggctcac gctgtaatcc caacactttg ggaggccgag
5400

gcgggtggat cacctgaggt caagagttca agaccagcct ggccaacatg gtgaaacccc
5460

aactctatta aaagtacaaa aaattagctg ggcatggtgg tgaacgcctg taaccccagc
5520

tacttgggag gctgaggcag gagaatcgct tgaacccagg aggtggaagt ttcagtgagc
5580

tgagatcacg ccattgcact ctagcctggg caacaaaaga gaaactccat ctcaaaaaaa
5640

aaaacaagga aaaaaagaaa cagccctcat gacacttaga aagtagaata gctggctgtt
5700

atctgaacat tgaattgtaa ggcttatcag gtggactttg cattccatca gcagacaatt
5760

tttttttttt tttttttttg agatggagtc tcattctgtc tcccaggctg gagggcagtg
5820

gtgcgatctc ggctcactgc aagctccacc tcctgggttc atgccattct cctgcctcag
5880

cctcccaagt agctgggacc acaggcaccc gccaccatgc ccagttaatt ttttgtattt
5940

ttagtagaga cggggtttca ccatgttagc caagatggtc tcgatctcct gacctcgtga
6000

tccgcccacc tcggcctccc aaagtgctgg gattacaggc atgagccacc gcgcctagcc
6060

tacaaatgtt ttgtaatagc tcttgaggcc catcttggag ttctcctttt gctaaaacca
6120

ctgaactctc taggaggaaa aaggaacttg gttcttgaca tatgtgtgca tgtatttcca
6180

tataaccttt aggaagctat tgcaatggta ctataaacta gaattttaga agatagaagg
6240

aaaatattct ggagatcatt gaagagaaat ggagtccaac actagttaaa gatgatgaag
6300

acagattttt ttttttgacg gagtctcgct ctgtcgccca ggctggagtg cagtggcaca
6360

atctcagctc actgcaaccc tccacctctt gggttcaagt gattctcctg cctcagcctc
6420

ccaagtagct gggactacag gcgcacacca ccacgcccgg ctaatttttg tatttttagt
6480

agagacaagg tttcaccata ttcgccaggc tggtctcgaa ctcctgacct tgtaatccgc
6540

ccaccttggc ctcccaaagt gctgggatta caggcatgag ccaccacgcc cggccgatga
6600

agacagattt tattcagtac taccacagta gaggaaagag ccaagttcaa ttccaaatac
6660

aacaaagaca ggtggagatt tatagccaat gagcagattg agggggtcag tggatggaat
6720

atttaagaag acatcaaggg tagggagctt cttgctaaag cttcatgtac ttaaacaaga
6780

agggtggggg atgagggaaa ttgatcagat atcaatggtg gcagtattga cttagcagga
6840

ttcttgctaa gaggtcttgc taggacagac ataggaagcc aaggtggagg tctagtcgaa
6900

aagaaggctc atcagagaag tctaactaaa gtttggtcaa gaagagtctt tgtcaaggta
6960

aatctatcat ttccctcaaa aggtaatttt caggatccca tcaggaagat tagcatggct
7020

gctagctttc tcctcagttc tgggctatag ctcacatgcc tagtttgaac tagctcagca
7080

gaactggggg atttattctt tgtcttccaa caaactcatc tggatgattt tgggggtttg
7140

tggggaaaag cccccaatac ctggtgaagt aaccttgtct cttcccccag cctggaatgg
7200

ttctctcttt ctgctacctc acgattgtgc ttctacaatg gtgactcttt tcctccctct
7260

catttcaggt tcacagcaaa ggactgaagc tagggattta tgcagatgtt ggaaataaaa
7320

cctgcgcagg cttccctggg agttttggat actacgacat tgatgcccag acctttgctg
7380

actggggagt agatctgcta aaatttgatg gttgttactg tgacagtttg gaaaatttgg
7440

cagatggtaa tgtttcattc cagagattta gccacaaagg aaagaacttt gaggccatgg
7500

tagctgagcc aaagaaccaa tcttcagaat tttaaatacc ctgtcacaat actggaaata
7560

attattctcc atgtgccaga gctcccatct cttctctttc agttcattaa ttaattaatt
7620

aattcatgta aaatccatgc atacctaacc atagctaata ttgtgcactt ataattcaag
7680

agggctctaa gagttaatta gtaattgtaa ctctctataa catcatttag gggagtccag
7740

gttgtcaatc ggtcacagag aaagaagcat cttcattcct gcctttcctc aatatacaca
7800

ccatctctgc actacttcct cagaacaatc ccagcagtct gggaggtact ttacacaatt
7860

taagcacaga gcaactgcct gtccctgctg ctagtttaaa catgaacctt ccaggtagcc
7920

tcttcttaaa atatacagcc ccagctgggc atgatggctc atgcctgtaa tcctagcact
7980

ttgggaggct gaggcgggtg gattacttga ggtcaggagt tcgagaccac cctggccaac
8040

atggtgaaac cccatctcta gtaaaaatac aaaaattagc tgactttggt ggcacatgcc
8100

tgtaatccca gctacttggg aagctgagac agaagagtca cttgaacctg ggaaacagag
8160

gttgcagtga gccaagatcg caccactgca ctccaccctg gatgacagac tgaaccccat
8220

ctcaaaaaat taaaataaaa taaaataaaa taactatata tatagcccca gctggaaatt
8280

catttctttc ccttatttta cccattgttt tctcatacag gttataagca catgtccttg
8340

gccctgaata ggactggcag aagcattgtg tactcctgtg agtggcctct ttatatgtgg
8400

ccctttcaaa aggtgagata gtgagcccag aatccaatag aactgtactg atagatagaa
8460

cttgacaaca aaggaaacca aggtctcctt caaagtccaa cgttacttac tatcatccta
8520

ccatctctcc caggttccaa ccacttctca ccatccccac tgctgtaatt atagcctaag
8580

ctaccatcac ctggaaagtc atccttgtgt cttccccttt atttcaccat tcatgtcctg
8640

tctatcaaca gtccttccac cagtatctct aaaatatctc ctgaatcagc ccacttcctt
8700

ccatcttcac tacatgcacc ctggccttcc aagctactat cggctctcaa ccagactgct
8760

gggaccacct gatctctctg cttccactct gtctcaaccc ccatctattt tccaagcagc
8820

actagagtta tcatattaaa atgtaaatat cagttttttt tttaaagaaa aaaaccctga
8880

gacttaacag agttataaaa aatataaatg tcatcatcag ttccctgctt aaaaccctta
8940

actcgcttcc aattgcactt ggaatgaaac caaactgcac tgatccagcc cttgcctgcc
9000

tccccaaagt ccaaggggtc atggctcttt ccctggctac actggttttc tttctgtccc
9060

tcaacactgc aagcctattg ctgccccagg gcctttacac ttgctttttt tctgcctaga
9120

acagttcttc cccaaagatt tttaaagggc cgggctcctt aacattgaag tcgcagacca
9180

aacgccacat atgcagacag ttcttctcta actactttaa aatagccctc tgtccattca
9240

ttcttcatca cattaacctg tttaattttc ttctcagagc tccacactat ttggaagtat
9300

ttgttgactt gttaccatgt ctccccacta gagtgtaagt ttcatgaggg cagggacctt
9360

gtctgacttt gactgtatct ctcgcatatg gttaagtgtt aaatagttat ttatggaatg
9420

aatccctatt attccctcat tatctctgca aaatagtctt ttttctcaac atcttaaacc
9480

tgatatccca cctgcctatc tacaaacttt ttttttgcga cagagtctca ctgtcaccca
9540

ggctagagtg cagtggcgcc atctcggctc actgcaacct ccgcctcccg ggtttaagcg
9600

attctcttgc ctcagcctcc cagtagctgg gattataggc gtgcgctacc acatctggct
9660

aatttttgta tttttagtag agatggtttc accatgttgg ccaggcttgt ctcgaactcc
9720

tgacctcaga tgatccacct gcctcggcct cccaaagtgc tgggattaca ggcatgagcc
9780

accgtgccca gcctctacaa actttttatt ccattaacaa actatatgct gggatttaag
9840

ttttcttaat acttgatgga gtcctatgta attttcgagc ttttaatttt actaagacca
9900

ttttagttct gattatagaa gtaaattaac tttaagggat ttcaagttat atggcctact
9960

tctgaagcaa acttcttaca gtgaaaattc attataaggg tttagacctc cttatggaga
10020

cgttcaatct gtaaactcaa gagaaggcta caagtgcctc ctttaaactg ttttcatctc
10080

acaaggatgt tagtagaaag taaacagaag agtcatatct gttttcacag cccaattata
10140

cagaaatccg acagtactgc aatcactggc gaaattttgc tgacattgat gattcctgga
10200

aaagtataaa gagtatcttg gactggacat cttttaacca ggagagaatt gttgatgttg
10260

ctggaccagg gggttggaat gacccagata tggtaaaaac ttgagccctc cttgttcaag
10320

accctgcggt aggcttgttt cctattttga cattcaaggt aaatacaggt aaagttcctg
10380

ggaggaggct ttatgtgaga gtacttagag caggatgctg tggaaagtgg tttctccata
10440

tgggtcatct aggtaacttt aagaatgttt cctcctctct tgtttgaatt atttcattct
10500

ttttctcagt tagtgattgg caactttggc ctcagctgga atcagcaagt aactcagatg
10560

gccctctggg ctatcatggc tgctccttta ttcatgtcta atgacctccg acacatcagc
10620

cctcaagcca aagctctcct tcaggataag gacgtaattg ccatcaatca ggaccccttg
10680

ggcaagcaag ggtaccagct tagacaggta aataagagta tatattttaa gatggcttta
10740

tatacccaat accaactttg tcttgggcct aaatctattt ttttcccttg ctcttgatgt
10800

tactatcagt aataaagctt cttgctagaa acattacttt atttccaaaa taatgctaca
10860

ggatcatttt aatttttcct acaagtgctt gatagttctg acattaagaa tgaatgccaa
10920

actaacaggg ccacttatca ctagttgcta agcaaccaca ctttcttggt ttttcaggga
10980

gacaactttg aagtgtggga acgacctctc tcaggcttag cctgggctgt agctatgata
11040

aaccggcagg agattggtgg acctcgctct tataccatcg cagttgcttc cctgggtaaa
11100

ggagtggcct gtaatcctgc ctgcttcatc acacagctcc tccctgtgaa aaggaagcta
11160

gggttctatg aatggacttc aaggttaaga agtcacataa atcccacagg cactgttttg
11220

cttcagctag aaaatacaat gcagatgtca ttaaaagact tactttaaaa tgtttatttt
11280

attgccaact actacttcct gtccaccttt ttctccattc actttaaaag ctcaaggcta
11340

ggtggctcat gcctgtaatc ccagcacttt gggaggctga ggcgggcaga tcacctgagg
11400

tcgggacttt gagacccgcc tggacaacat ggtgaaaccc catttctaat aaaaatataa
11460

aaattagcca ggtgtggtgg cgcacctgtg gtcccagcta ctctgggggc tgaggcatga
11520

gaatcgcttg aacccgggag tggaggttgc attgagctga gatcatgcca cctcactcca
11580

gcctgggcaa caaagattcc atctcaaaaa aaaaaaaaaa gccaggcaca gtggctcatg
11640

cctggaatcc cagcactttt ggaagctgag gcaggcagat cacttgaggt taggatttca
11700

agaccagcct ggctaacata gtaaagccct gtctctacta aaaatacaaa aattagccag
11760

gtatggtggc gagcttctgt agccccagct actcaggaga ctgaggcagg agaatcactt
11820

gaacccggga agtggggggg tgcagtgacc caagatcacg ccactgcatt ccagcctggg
11880

caacagagca agactccatc tcaaaaaaaa aagttctatt tccttgaata aaattttccg
11940

aagtttaaac tttaggaata aaactattaa acccgtattt actcatccag atacccaccc
12000

cccttgttga gattctctcc caattatcaa aatgtgtagc atatttaact accaagagct
12060

aaacatcatt aagactgaaa tgtattaaga aggatgtata ggccaggcac ggtgtctcac
12120

gcctgtaatc ccaacacttt gggaggccaa gtcgggcgga tcacgaggtc aggagatgga
12180

gaccatcctg gccaacatgg tgaaaccccc tctctactaa aaatacaaaa attagccagg
12240

caggtggcag gcacctgtaa tcccagctac tccagaggct gaggcaggac aatcacttga
12300

acctgggagg cagaggctgc agtgagctga ggttgtacca attgcactcc agcctaggta
12360

acgagcaaca ctccatctca aaaaaagaaa aaaaaaaaga tgtataattt ggaactgtta
12420

agaggcattt taaaga
12436

<210> 2
<211> 429
<212> PRT
<213> Homo sapiens

<400> 2

<210>  3
<211>  1290
<212>  DNA
<213>  Homo sapiens

<400>  3
atgcagctga ggaatcccga gctccacctg ggctgtgctc tggctctgcg gttcctggcc
60

ctcgtgtcct gggacatccc tggcgctagg gccctcgata acggactggc ccggaccccc
120

acaatgggat ggctccactg ggaaaggttc atgtgcaatc tggactgtca ggaggaaccc
180

gactcctgca tcagcgaaaa gctcttcatg gagatggccg agctgatggt gagcgagggc
240

tggaaggacg ccggctacga gtatctgtgc atcgatgact gctggatggc ccctcaaagg
300

gactccgaag gcaggctgca ggctgatccc caaaggtttc cccacggaat ccggcagctc
360

gccaactacg tgcattccaa gggcctcaag ctcggcatct acgccgacgt gggcaacaaa
420

acatgcgccg gattccccgg cagcttcggc tactacgaca tcgacgccca gacattcgct
480

gattggggag tggacctgct gaagttcgac ggctgttact gcgattccct ggaaaacctg
540

gccgacggct acaaacacat gtccctcgcc ctgaaccgga caggcaggtc catcgtgtac
600

agctgcgagt ggcccctgta catgtggcct ttccagaagc ccaactacac agagatcagg
660

cagtactgca accactggag gaacttcgct gacatcgacg actcctggaa gagcatcaag
720

agcatcctgg actggaccag cttcaaccag gagaggatcg tggacgtggc tggacccgga
780

ggctggaacg accccgatat gctggtgatt ggcaacttcg gactgagctg gaaccagcag
840

gtgacccaga tggccctgtg ggccattatg gccgctcccc tgttcatgtc caacgacctg
900

aggcacatca gcccccaggc caaggctctg ctgcaggaca aggatgtgat cgccatcaac
960

caggaccccc tgggcaagca gggctaccag ctgaggcaag gagataactt cgaggtgtgg
1020

gagaggcccc tgtccggact ggcttgggcc gtggccatga tcaatcggca ggagatcggc
1080

ggaccccggt cctacaccat tgctgtggcc agcctgggaa aaggagtcgc ctgcaacccc
1140

gcctgcttca ttacccagct gctccccgtg aagcggaagc tgggcttcta tgagtggacc
1200

agcaggctga ggtcccatat caatcctacc ggcaccgtcc tcctccagct cgagaatacc
1260

atgcagatga gcctcaagga tctgctgtga
1290